# EUROPEAN PATENT SPECIFICATION

(11) **EP 4 509 493 B1**
(45) Date of publication and mention of the grant of the patent: **02.09.2026**
(21) Application number: 23807489.2
(22) Date of filing: 09.05.2023
(51) Int. Cl.: C07C 231/12, C07C 233/18, C09K 3/18, D06M 15/263, C08F 2/22, C08F 2/38, C08F 4/04, C08F 220/18, C08F 220/38, C09D 5/16, D06M 101/34, D06N 3/00, D06N 3/14, C08F 220/36, D06M 15/285, D06M 101/32

(54) **PRODUCTION METHOD FOR AMIDE GROUP-CONTAINING MONOMER**
HERSTELLUNGSVERFAHREN FÜR AMIDGRUPPENHALTIGES MONOMER
PROCÉDÉ DE PRODUCTION D'UN MONOMÈRE CONTENANT UN GROUPE AMIDE

(30) Priority: 18.05.2022 JP 2022081699
(43) Date of publication of application: 19.02.2025
(73) Proprietor: DAIKIN INDUSTRIES, LTD., Osaka-shi, Osaka 530-0001 (JP)
(72) Inventor: UESUGI, Norimasa, Osaka-Shi, Osaka 530-0001 (JP); SAKASHITA, Hirotoshi, Osaka-Shi, Osaka 530-0001 (JP)
(74) Representative: Hoffmann Eitle
(86) International application number: PCT/JP2023/017397
(87) International publication number: WO 2023/223885

(56) References cited:
- EP-A2- 0 448 399
- JP-A- 2001 270 856
- JP-A- 2001 509 200
- JP-A- 2020 128 529
- HE JUAN ET AL: "The conversion of castor oil to a series of functional polyamides inspired by natural silks", INDUSTRIAL CROPS AND PRODUCTS, vol. 181, 30 March 2022 (2022-03-30), NL, pages 114852, XP093283917, ISSN: 0926-6690, DOI: 10.1016/j.indcrop.2022.114852
- OGURA YUSUKE, ARTAR MÜGE, PALMANS ANJA R. A., SAWAMOTO MITSUO, MEIJER E. W., TERASHIMA TAKAYA: "Self-Assembly of Hydrogen-Bonding Gradient Copolymers: Sequence Control via Tandem Living Radical Polymerization with Transesterification", MACROMOLECULES, AMERICAN CHEMICAL SOCIETY, US, vol. 50, no. 8, 25 April 2017 (2017-04-25), US , pages 3215 - 3223, XP093108798, ISSN: 0024-9297, DOI: 10.1021/acs.macromol.7b00070

## Description

### Technical Field

The present disclosure relates to a production method for an amide group-containing monomer.

### Background Art

Patent Literature 1 discloses as a water-repellent composition imparting excellent water-repellency, a water-repellent composition for use, containing a fluorine-containing polymer obtained by polymerizing a monomer having an amide structure represented by the formula:

R₂C(=O)NHR³OR¹

wherein
R¹ is an organic residue having an ethylenically unsaturated polymerizable group,
R² is a hydrocarbon group having 7 to 30 carbon atoms, and
R³ is a hydrocarbon group having 1 to 5 carbon atoms.

Patent Literature 2 discloses a method for the synthesis of heptadecyl carbamido ethyl acrylate (HDCMEA) monomer from octadecanoyl chloride, ethanolamine and acryloyl chloride.

### Citation List

### Patent Literature

Patent Literature 1: JP 6737417 B
Patent Literature 2: EP 0448399 A2

### Summary of Invention

The invention is defined in the claims. Any subject-matter which is not encompassed by the claims is not part of the invention and provided for reference or comparison purposes only.

### Technical Problem

An object of the present disclosure is to provide a method for efficiently producing an amide group-containing monomer with few impurities. The other object is also to provide a method for producing an amide group-containing polymer with excellent standing stability, excellent workability and productivity.

### Solution to Problem

The present disclosure includes the following embodiments:
[Item 1] A method for producing an amide group-containing monomer comprising:
   (I) reducing amounts of Na, K, and Li present as impurities in an amide group-containing alcohol (2) of the formula HO-Y¹. NHC(=O) -R¹² wherein Y¹ and R¹² are as defined below;
   (II) conducting a transesterification reaction to obtain an amide group-containing monomer represented by the following formula:

      CH₂=C(-R¹¹)C(=O)O-Y¹-NHC(=O)-R¹²
   wherein
   R¹¹ is H, halogen or a monovalent organic group selected from the group consisting of a methyl group, a cyano group, a substituted or unsubstituted benzyl group, or a substituted or unsubstituted phenyl group,
   Y¹ is a divalent hydrocarbon group having 1 to 6 carbon atoms, and
   R¹² is a monovalent hydrocarbon group having 6 to 40 carbon atoms, from the amide group-containing alcohol (2),
   wherein a total amount of Na, K, and Li present as impurities in the amide group-containing alcohol is 300 ppm or less.
[Item 2] The method for producing an amide group-containing monomer according to item 1, wherein a total amount of alkali metal elements present as impurities in the amide group-containing alcohol is 300 ppm or less.
[Item 3] The method for producing an amide group-containing monomer according to item 1 or 2, wherein reducing amounts of Na, K, and Li present as impurities in the amide group-containing alcohol is conducted by water washing or alkali adsorption treatment.
[Item 4] The method for producing an amide group-containing monomer according to any one of items 1 to 3, wherein in the amide group-containing alcohol, R¹² is an aliphatic hydrocarbon group having 11 or more carbon atoms.
[Item 5] The method for producing an amide group-containing monomer according to any one of items 1 to 4, wherein in the amide group-containing alcohol,
   R¹¹ is a hydrogen atom or a methyl group,
   Y¹ is an alkylene group having 2 to 4 carbon atoms, and
   R¹² is a linear alkyl group having 10 to 30 carbon atoms.
[Item 6] The method for producing an amide group-containing monomer according to any one of items 1 to 5, wherein a total amount of Na, K and Li present as impurities in the amide group-containing alcohol is 30 ppm or less.
[Item 7] The method of any one of items 1 to 6, wherein an amount of a Michael addition product present as an impurity in the amide group-containing monomer (1) is 2 wt. % .
[Item 8] The amide group-containing monomer according to any one of items 1 to 7, wherein R¹² is an aliphatic hydrocarbon group having 11 or more carbon atoms.
[Item 9] The amide group-containing monomer according to any one of items 1 to 8, wherein
   R¹¹ is a hydrogen atom or a methyl group,
   Y¹ is an alkylene group having 2 to 4 carbon atoms, and
   R¹² is a linear alkyl group having 10 or more and 30 or less carbon atoms.
[Item 10] A method for producing an amide group-containing polymer, comprising polymerizing the amide group-containing monomer obtained by the method of any one of items 1-9.
[Item 11] The method according to item 10, wherein in the polymerizing, at least one selected from the group consisting of a hydrocarbon-based monomer, a crosslinkable monomer, and a halogenated olefin is co-polymerized.
[Item 12] A method for producing a dispersion, comprising producing an amide group-containing polymer by the method according to item 10 or 11.
[Item 13] A method for producing a composition which is a water-repellent composition or an oil-resistant agent composition, comprising producing an amide group-containing polymer by the method according to item 10 or 11.
[Item 14] A method for producing a treated textile product or paper product, comprising: producing a composition by the method according to item 13; and treating a substrate with said composition.

### Advantageous Effects of Invention

According to the method for producing an amide group-containing monomer in the present disclosure, an amide group-containing monomer with few impurities can be effectively produced. Moreover, a dispersion produced using the amide group-containing monomer obtained by the method for producing an amide group-containing monomer in the present disclosure has excellent standing stability and is advantageous from the viewpoints of workability and productivity.

### Brief Description of Drawings

[Figure 1] Figure 1 is a schematic diagram of a sedimentation tube used in a spontaneous sedimentation test of Example of the present disclosure.
[Figure 2] Figure 2 is a schematic diagram of a sedimentation tube used in a centrifugal sedimentation test of Example of the present disclosure.

### Description of Embodiments

### <Amide group-containing monomer>

The amide group-containing monomer in the present disclosure is represented by the following formula:

CH₂=C(-R¹¹)C(=O)O-Y¹-NHC(=O)-R¹²

wherein
R¹¹ is H, halogen or a monovalent organic group selected from the group consisting of a methyl group, a cyano group, a substituted or unsubstituted benzyl group,
or a substituted or unsubstituted phenyl group,
Y¹ is a divalent hydrocarbon group having 1 to 6 carbon atoms, and
R¹² is a monovalent hydrocarbon group having 6 to 40 carbon atoms,
wherein an amount of a Michael addition product present as an impurity in the amide group-containing monomer is less than 2% by weight relative to the amide group-containing monomer.

R¹¹ is a hydrogen atom, a monovalent organic group or a halogen atom. The monovalent organic group is selected from the group consisting of a methyl group, a cyano group, a substituted or unsubstituted benzyl group and a substituted or unsubstituted phenyl group. Examples of the halogen atoms include fluorine, chlorine, bromine, iodine, and the like. R¹¹ is preferably a hydrogen atom, a methyl group, or a chlorine atom, and particularly a hydrogen atom or a methyl group.

Y¹ is a divalent hydrocarbon group having 1 to 6 carbon atoms. The number of carbon atoms of Y¹ may be 1 to 6, for example, 1 or more, 2 or more, 3 or more, or 4 or more, and may be 6 or less, 5 or less, 4 or less, or 3 or less, and it is for example, 2 to 4, or 2 to 3. Y¹ may be an aliphatic hydrocarbon group or aromatic hydrocarbon group, and is preferably an aliphatic hydrocarbon group, for example an alkylene group. Y¹ may be linear or branched, and is preferably linear.

R¹² is a monovalent hydrocarbon group having 6 to 40 carbon atoms. R¹² is preferably a branched or long chain (or long chain linear) hydrocarbon group. The hydrocarbon group may be an aliphatic hydrocarbon or aromatic hydrocarbon group, and is preferably an aliphatic hydrocarbon group, and particularly a saturated aliphatic hydrocarbon group (alkyl group). The number of carbon atoms of R¹² may be 6 or more, 8 or more, 10 or more, 11 or more, 12 or more, 14 or more, 16 or more, or 18 or more, and is preferably 10 or more and more preferably 12 or more. The number of carbon atoms of R¹¹ may be 40 or less, 35 or less, 30 or less, 25 or less, 20 or less, 15 or less, or 10 or less, and is preferably 30 or less and more preferably 25 or less.

Specific examples of the amide group-containing monomers are as follows. Each of the compounds of the following chemical formulas is an acrylic compound having a hydrogen atom at the α-position, and may also be a methacrylic compound having a methyl group at the α-position and an α-chloroacrylic compound having a chlorine atom at the α-position.

CH₂=CHC(=O)OCₘH₂ₘNHC(=O)CₙH₂ₙ₊₁

CH₂=CHC(=O)OC₂H₄NHC(=O)C₁₇H₃₅

CH₂=CHC(=O)OC₂H₄NHC(=O)C₁₅H₃₁

and
a mixture of CH₂=CHC(=O)OC₂H₄NHC(=O)C₁₇H₃₅ and CH₂=CHC(=O)OC₂H₄NHC(=O)C₁₅H₃₁
wherein n is 6 to 40 and m is 1 to 6.

### [Michael addition product]

The amide group-containing monomer can have a Michael addition product as an impurity. The Michael addition product has a structure in which an alcohol is added to the amide group-containing monomer, and may have a structure in which an alcohol is added to a dimer of the amide group-containing monomer. Herein, the alcohol may be an amide group-containing alcohol that is the amide group-containing monomer, or may be an alcohol generated by decomposition of raw material ester.

Examples of the Michael addition products having a structure in which an alcohol is added to the amide group-containing monomer include compounds represented by the formulas:

R¹²-C(=O)NH-Y¹-O-CH₂CH(-R¹¹)C(=O)O-Y¹-NHC(=O)-R¹²

R¹³-O-CH₂CH(-R¹¹)C(=O)O-Y¹-NHC(=O)-R¹²

and specific examples thereof include 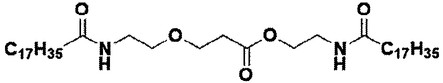 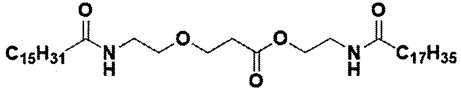 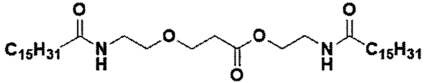 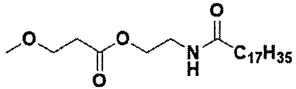 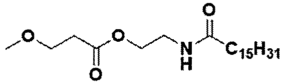 and the like.

Examples of the Michael addition products having a structure in which an alcohol is added to a dimer of the amide group-containing monomer include compounds represented by the formulas:

R¹²-C(=O)NH-Y¹-O-CH₂C(CH₂CH(-R¹¹)C(=O)O-Y¹-NHC(=O)-R¹²)(-R¹¹)C(=O)O-Y¹-NHC(=O)-R¹²

R¹³-O-CH₂C(CH₂CH(-R¹¹)C(=O)O-Y¹-NHC(=O)-R¹²)(-R¹¹)C(=O)O-Y¹-NHC (=O) -R¹²

and specific examples thereof include 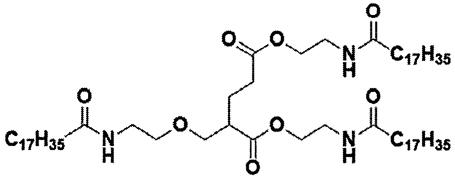 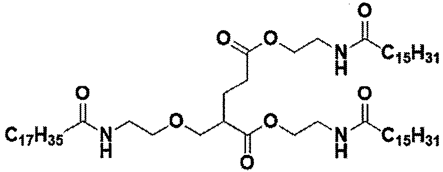 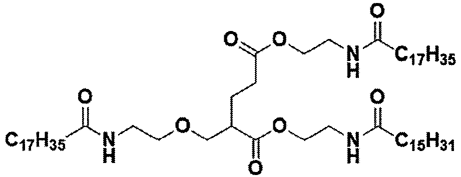 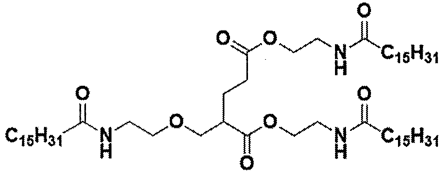 and the like.

The amount of the Michael addition product in the amide group-containing monomer may be 0% by weight or more, 0.1% by weight or more, 0.3% by weight or more, 0.5% by weight or more, or 0.7% by weight or more, relative to the amide group-containing monomer. The amount of the Michael addition product in the amide group-containing monomer may be 2% by weight or less, less than 2% by weight, 1.6% by weight or less, 1.2% by weight or less, 0.8% by weight or less, or 0.4% by weight or less, and is preferably 1.2% by weight or less and more preferably 0.8% by weight or less, relative to the amide group-containing monomer. The content of the Michael addition product can be measured by a general method such as LCMS analysis.

### <Method for producing amide group-containing monomer>

The method for producing an amide group-containing monomer in the present disclosure includes conducting a transesterification reaction to obtain an amide group-containing monomer represented by the following formula:

CH₂=C(-R¹¹)C(=O)O-Y¹-NHC(=O)-R¹²

wherein
R¹¹, Y¹ and R¹² are as defined in the claims, from an amide group-containing alcohol represented by the following formula:

HO-Y¹-NHC(=O)-R¹²

wherein a total amount of Na, K, and Li present as impurities in the amide group-containing alcohol is 300 ppm or less.

### [Transesterification reaction step]

The method for producing an amide group-containing monomer in the present disclosure includes a transesterification reaction. In the transesterification reaction, the amide group-containing alcohol and a raw material ester are reacted using a catalyst, if necessary, to be able to obtain the amide group-containing monomer.

### (Amide group-containing alcohol)

The amide group-containing alcohol in the present disclosure is represented by the following formula:

HO-Y¹-NHC(=O)-R¹²

wherein
Y¹ and R¹² are as defined in the claims, and
wherein the total amount of Na, K, and Li present as impurities is 300 ppm or less.

The amide group-containing alcohol is a compound in which CH₂=C(-R¹¹)C(=O)- in the amide group-containing monomer is replaced by H-, and Y¹ and R¹² explained in the amide group-containing monomer can be incorporated as Y¹ and R¹² explained in the amide group-containing alcohol.

### (Alkali metal element)

The amide group-containing alcohol may have an alkali metal element as an impurity, particularly at least one selected from the group consisting of Na, K, and Li. The alkali metal element may be present in the form of a salt, oxide, hydroxide, alcoholate, or ion. According to the invention, the total amount of Na, K, and Li present as impurities in the alcohol (2) is ≤ 300 ppm.

The total amount of the alkali metal elements in the amide group-containing alcohol may be 0 ppm or more, 5 ppm or more, 30 ppm or more, 50 ppm or more, 70 ppm or more, or 100 ppm or more. In the amide group-containing alcohol, the total amount of alkali metal elements may be 300 ppm or less, less than 300 ppm, 280 ppm or less, 200 ppm or less, 160 ppm or less, 120 ppm or less, 80 ppm or less, 50 ppm or less, 30 ppm or less, or 10 ppm or less, and is preferably 200 ppm or less, more preferably 50 ppm or less, and particularly preferably 30 ppm or less.

The total amount of Na, K and Li in the amide group-containing alcohol may be 0 ppm or more, 5 ppm or more, 30 ppm or more, 50 ppm or more, 70 ppm or more, or 100 ppm or more. The total amount of Na, K and Li in the amide group-containing alcohol may be 300 ppm or less, less than 300 ppm, 280 ppm or less, 200 ppm or less, 160 ppm or less, 120 ppm or less, 80 ppm or less, 50 ppm or less, 30 ppm or less, or 10 ppm or less, and is preferably 200 ppm or less, more preferably 50 ppm or less, and particularly preferably 30 ppm or less. The total amount of alkali metal elements and the total amount of Na, K and Li can be measured by ICP atomic emission spectroscopy or the like.

### (Raw material ester)

The raw material ester is represented by the following formula:

CH₂=C(-R¹¹)C(=O)OR¹³

wherein R¹¹ and R¹³ are as defined hereinabove.

The raw material ester is a compound in which -O-Y¹-NHC(=O)-R¹² in the amide group-containing monomer is substituted with -OR¹³, and R¹¹ explained in the amide group-containing monomer can be incorporated as R¹¹ explained in the raw material ester. R¹¹ in the raw material ester is, for example, hydrogen or a methyl.

R¹³ is a monovalent organic group, and is not limited as long as it is a group that does not lose transesterification reactivity between the raw material ester and an alcohol, but is typically a hydrocarbon group, and may be an aliphatic hydrocarbon group having 1 to 10 carbon atoms, for example, an alkyl group having 1 to 6 carbon atoms (for example, 1 to 3 carbon atoms), and particularly a methyl group or ethyl group.

A molar ratio of the raw material ester used in the transesterification reaction to the amide group-containing alcohol is usually 1 or more. The molar ratio of the raw material ester to the amide group-containing alcohol may be 1.1 or more, 2 or more, or 3 or more. The molar ratio of the raw material ester to the amide group-containing alcohol may be 10 or less, 8 or less, or 6 or less.

### (Catalyst)

A catalyst may be used for the transesterification reaction, if necessary. Examples of the catalysts are not limited, and known transesterification catalysts can be used, and the catalyst may be a homogeneous catalyst or a heterogeneous catalyst. Examples of the catalysts include oxides, such as barium oxide, lead oxide, zinc oxide, and zirconium oxide; hydroxides, such as tin hydroxide, lead hydroxide and nickel hydroxide; chlorides such as tin chloride, lead chloride, zirconium chloride, and nickel chloride; carbonates, such as lead carbonate, zinc carbonate, nickel carbonate, and rubidium carbonate; hydrogen carbonates such as rubidium hydrogen carbonate and cesium hydrogen carbonate; phosphates, such as rubidium phosphate, lead phosphate, zinc phosphate, and nickel phosphate; nitrates, such as lead nitrate, zinc nitrate, and nickel nitrate; acetates, such as lead acetate, zinc acetate, and nickel acetate; alkoxy compounds such as tetramethoxytitanium, tetraethoxytitanium, tetrabutoxytitanium, tetraisopropoxytitanium, tetrapropoxyzirconium, and tetrabutoxyzirconium; acetylacetonate complex compounds such as zinc acetylacetonate, nickel acetylacetonate, dibutoxytitanium acetylacetonate, titanium acetylacetonate, magnesium acetylacetonate, and dibutoxytin acetylacetonate; tin compounds such as dimethyltin oxide, dibutyltin oxide, dibutyltin acetate, and dibutyltin dilaurate; chelate compounds such as titanium triethanolaminate (titanium (IV) bis(triethanolaminate)diisoproxide); and the like. The amount of the catalyst used is in the range of 0.02 to 10% by weight and more preferably 0.1 to 5% by weight, relative to the total amount of the raw material ester and the amide group-containing alcohol.

### (Polymerization inhibitor)

From the viewpoint of inhibiting polymerization of the raw material ester and the amide group-containing monomer of a product, a polymerization inhibitor is preferably used. As the polymerization inhibitor, any known and commonly used polymerization inhibitor for polymer synthesis can be used without any particular limitations. Examples of the polymerization inhibitor include benzoquinone, hydroquinone, catechol, diphenylbenzoquinone, hydroquinone monomethyl ether, naphthoquinone, t-butylcatechol, t-butylphenol, dimethyl-t-butylphenol, t-butylcresol, phenothiazine, and the like, and these may be used singly or in combinations of two or more thereof.

The amount of the polymerization inhibitor can be appropriately set according to the reaction conditions or the like. The amount of polymerization inhibitor may be usually 5 ppm or more, 10 ppm or more, 50 ppm or more, 100 ppm or more, or 300 ppm or more, relative to a polymerizable reaction raw material. The amount of polymerization inhibitor may be 10,000 ppm or less, 5,000 ppm or less, 1,000 ppm or less, or 500 ppm or less, relative to a polymerizable reaction raw material.

### (Reaction conditions)

The transesterification reaction can be carried out in the absence of a solvent or in the presence of a suitable solvent that does not affect a reactant of the transesterification reaction.

A temperature in the transesterification reaction can be appropriately set according to a reaction raw material, a reaction solvent, or the like. In general, the higher the reaction temperature, the faster the reaction rate. The reaction temperature in the transesterification reaction may be 20°C or higher, 30°C or higher, 60°C or higher, or 80°C or higher. The reaction temperature in the transesterification reaction may be 180°C or lower, 160°C or lower, 140°C or lower, 120°C or lower, or 100°C or lower.

A reaction time can be appropriately set depending on the reaction temperature, reaction raw material, or the like. The reaction time may be 15 minutes or longer, 30 minutes or longer, 1 hour or longer, or 3 hours or longer. The reaction time may be 48 hours or shorter, 24 hours or shorter, 12 hours or shorter, 6 hours or shorter, or 3 hours or shorter.

The transesterification reaction step in the present disclosure is outlined as follows. A reactor (for example, a vessel with a Dean-Stark tube) is charged with an amide group-containing alcohol and a raw material ester in an appropriate proportion. Next, appropriate amounts of a catalyst, a polymerization inhibitor, and a solvent are added to a reaction mixture, if necessary. The reaction mixture is heated to an appropriate temperature range, usually a reflux temperature of the reaction system, while the reaction mixture is stirred under air flow. While the reaction mixture is stirred, in order for completion of reaction, the alcohol resulting from the transesterification reaction is, in many cases, removed from a fractional distillation tube during the reaction as an azeotrope with an excess raw material ester or reaction solvent. At the same time, the same amount of raw material ester or reaction solvent may be added, if necessary, to the reaction mixture to keep the volume of the reactor contents constant. During the reaction, the content of the target product in the reaction mixture is tracked by gas chromatography analysis or the like, and the reaction may be continued until the content of the target product is 90% or more.

### [Reduction step]

The method for producing an amide group-containing monomer in the present disclosure includes the process step of reducing alkali metal elements selected from the group consisting of Na, K, and Li, present as impurities in an amide group-containing alcohol prior to a transesterification reaction. The reducing is not limited, but the amounts of Na, K, and Li present as impurities in the amide group-containing alcohol may be reduced by water washing or alkali adsorption treatment.

Water washing or an alkali adsorption treatment may be repeated, if necessary, until the total amount of Na, K and Li, reaches the above range. For the water washing, pure water is preferably used, for example, warm pure water may be used. The alkali adsorption treatment may be performed by bringing an amide group-containing alcohol solution into contact with an alkali adsorbent support. As the alkali adsorbent support, various adsorbent materials such as zeolite, metallosilicate, aluminum silicate, magnesium silicate, and activated carbon may be used. Generally, the larger the specific surface area of the adsorbent, the better the adsorption capability, but conversely, the more difficult it is to handle and remove the adsorbent. The specific surface area is not limited, but ought to be appropriately selected in full consideration of the above, and is preferably 50 m²/g to 800 m²/g.

### [Purification step]

The method for producing an amide group-containing monomer in the present disclosure may include purifying the amide group-containing monomer that is a transesterification reaction product. For example, the transesterification reaction product may be purified by column fractionation or the like, if necessary.

### <Amide group-containing polymer>

The amide group-containing polymer in the present disclosure has a repeating unit derived from the amide group-containing monomer described above. The amide group-containing polymer in the present disclosure may further have a repeating unit derived from at least one selected from the group consisting of a hydrocarbon-based monomer, a crosslinkable monomer, and a halogenated olefin. The amide group-containing polymer in the present disclosure may be free of a fluoroalkyl group having 8 or more carbon atoms, a perfluoroalkyl group having 8 or more carbon atoms, a perfluoroalkyl group, a fluoroalkyl group, or a fluorine atom.

### [Hydrocarbon-based monomer]

The hydrocarbon-based monomer has one ethylenically unsaturated double bond and a hydrocarbon group having 6 to 40 carbon atoms, provided that the above-described amide group-containing monomer is not included.

The hydrocarbon-based monomer may have at least one hydrocarbon group having 6 to 40 carbon atoms. It is preferable that the hydrocarbon group is an aliphatic hydrocarbon group, particularly a saturated aliphatic hydrocarbon group, and particularly an alkyl group. The hydrocarbon group may be linear or branched, and preferably linear. The number of carbon atoms of the hydrocarbon group may be 6 or more, 8 or more, 10 or more, 12 or more, 14 or more, 16 or more, or 18 or more, and is preferably 10 or more and more preferably 12 or more. The number of carbon atoms of the hydrocarbon group may be 40 or less, 35 or less, 30 or less, 25 or less, 20 or less, 15 or less, or 10 or less, and is preferably 30 or less and more preferably 25 or less.

Specific examples of the hydrocarbon-based monomers are as follows. Each of the compounds of the following chemical formulas is an acrylic compound having a hydrogen atom at the α-position, and may also be a methacrylic compound having a methyl group at the α-position and an α-chloroacrylic compound having a chlorine atom at the α-position. However, only compounds wherein the structural element corresponding to Y¹ is a divalent C₁₋₆ hydrocarbon group are part of the invention.

CH₂=CHC(=O)OCₙH2ₙ₊₁

CH₂=CHC(=O)OC₁₈H₃₇

CH₂=CHC(=O)OC₁₆H₃₃

CH₂=CHC(=O)OC₂H₄OC(=O)NHCₙH₂ₙ₊₁

CH₂=CHC(=O)OC₂H₄NHC(=O)OCₙH₂ₙ₊₁

CH₂=CHC(=O)OC₂H₄NHC(=O)NHCₙH₂ₙ₊₁

CH₂=CHC(=O)OC₄H₈OC(=O)NHCₙH₂ₙ₊₁

CH₂=CHC(=O)OC₂H₄OC(=O)NHC₁₈H₃₇

CH₂=CHC(=O)OC₂H₄NHC(=O)OC₁₈H₃₇

CH₂=CHC(=O)NHCₘH₂ₘOC(=O)NHCₙH₂ₙ₊₁

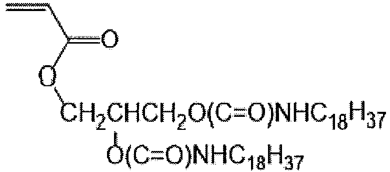

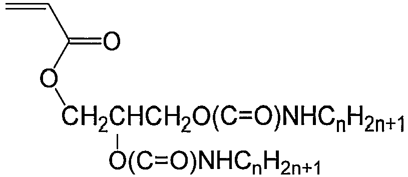

CH₂=CHC(=O)OCₘH₂ₘNHSO₂CₙH₂ₙ₊₁

CH₂=CHC(=O)OCₘH₂ₘSO₂NHCₙH₂ₙ₊₁

wherein n is 6 to 40, and m is 1 to 6.

From the viewpoint of liquid-repellency of the water-repellent composition, the hydrocarbon-based monomer may contain a hydrocarbon-based monomer having an amide group, a urea group, or a urethane group. Examples of hydrocarbon-based monomer having an amide group, urea group or urethane group include CH₂=C(-R²¹)-C(=O)-O-(CH₂)ₘ-O-C(=O)-NH-R²²,CH₂=C(-R²¹)-C(=O)-O-(CH₂)ₘ-NH-C(=O)-O-R²², and CH₂=C(-R²¹)-C(=O)-O-(CH₂)ₘ-NH-C(=O)-NH-R²². Herein, m may be an integer of 1 to 6, and particularly 2 or 4.

The hydrocarbon-based monomer may be a non-cyclic hydrocarbon group-containing monomer singly, but may contain a cyclic hydrocarbon group-containing monomer. Cyclic hydrocarbon group-containing monomer is a monomer having a cyclic hydrocarbon group and may be a monomer having one ethylenically unsaturated double bond and a cyclic hydrocarbon group.

Cyclic hydrocarbon group-containing monomer preferably has a (meth)acrylic group as an ethylenically unsaturated double bond, and for example, it may have a (meth)acrylate group or (meth)acrylamide group as an ethylenically unsaturated double bond.

The cyclic hydrocarbon group may be alicyclic or aromatic and is preferably alicyclic. The cyclic hydrocarbon group may be saturated or unsaturated and is preferably saturated. The cyclic hydrocarbon group may be a monocyclic group, polycyclic group, or bridged ring group and is preferably the bridged ring group. The cyclic hydrocarbon group may have a chain group (for example, linear or branched chain hydrocarbon group).

The number of carbon atoms of the cyclic hydrocarbon group may be 4 or more, 6 or more, or 8 or more, and may be 30 or less, 26 or less, 22 or less, 18 or less, or 14 or less.

Specific examples of the cyclic hydrocarbon groups include a cyclohexyl group, t-butylcyclohexyl group, adamantyl group, 2-methyl-2-adamantyl group, 2-ethyl-2-adamantyl group, bornyl group, isobornyl group, norbornyl group, dicyclopentanyl group, dicyclopentenyl group, benzyl group, phenyl group, naphthyl group, 2-t-butylphenyl group, a residual group formed by removing one or more hydrogen atoms from any of these groups (for example, a cyclohexylene group, adamantylene group, phenylene group, and naphthylene group), and a group formed by substituting any of these groups.

Specific examples of cyclic hydrocarbon group-containing monomer include cyclohexyl (meth)acrylate, t-butylcyclohexyl (meth)acrylate, benzyl (meth)acrylate, isobornyl (meth)acrylate, dicyclopentanyl (meth)acrylate, dicyclopentenyl (meth)acrylate, dicyclopentanyloxyethyl (meth)acrylate, tricyclopentanyl (meth)acrylate, adamantyl (meth)acrylate, 2-methyl-2-adamantyl (meth)acrylate, 2-ethyl-2-adamantyl (meth)acrylate, and a compound in which these acrylates are substituted with acrylamide. These may be used singly or in combination of two or more thereof.

### [Halogenated olefin monomer]

The amide group-containing polymer may have a repeating unit derived from a halogenated olefin monomer. The halogenated olefin monomer is preferably free of a fluorine atom. The halogenated olefin monomer is preferably an olefin having 2 to 20 carbon atoms, substituted with 1 to 10 chlorine atoms, bromine atoms or iodine atoms. The halogenated olefin monomer is preferably a chlorinated olefin having 2 to 20 carbon atoms and particularly preferably an olefin having 2 to 5 carbon atoms and having 1 to 5 chlorine atoms. Preferred specific examples of the halogenated olefin monomers are vinyl halides, such as vinyl chloride, vinyl bromide, and vinyl iodide, and vinylidene halides, such as vinylidene chloride, vinylidene bromide, and vinylidene iodide. Vinyl chloride is preferred because of its high water-repellency (particularly durability of water-repellency). The presence of repeating units derived from the halogenated olefin monomer enhances washing durability that the amide group-containing polymer imparts.

### [Crosslinkable monomer]

The amide group-containing polymer may have a repeating unit derived from a crosslinkable monomer. Crosslinkable monomer is a monomer capable of imparting crosslinkability to a polymer and may have at least two selected from the group consisting of a reactive group and an olefinic carbon-carbon double bond. Crosslinkable monomer may be a compound having at least two ethylenically unsaturated double bonds, or a compound having at least one ethylenically unsaturated double bond and at least one reactive group.

Crosslinkable monomer preferably has a (meth)acrylic group as an ethylenically unsaturated double bond, and for example, it may have a (meth)acrylate group or (meth)acrylamide group as an ethylenically unsaturated double bond.

Examples of the reactive groups include a hydroxyl group, epoxy group, chloromethyl group, blocked isocyanate group, amino group, carboxyl group, carbonyl group, and isocyanate group (block isocyanate group).

Specific examples of crosslinkable monomer include diacetone (meth)acrylamide, N-methylol (meth)acrylamide, hydroxyethyl (meth)acrylamide, glycidyl (meth)acrylate, hydroxymethyl (meth)acrylate, hydroxyethyl (meth)acrylate, 2,3-dihydroxypropyl (meth)acrylate, 3-chloro-2-hydroxypropyl (meth)acrylate, 2-acetoacetoxyethyl (meth)acrylate, butadiene, isoprene, chloroprene, vinyl monochloroacetate, vinyl methacrylate, glycidyl(meth)acrylate, 1,4-butanediol di(meth)acrylate, 1,6-hexanediol di(meth)acrylate, 1,9-nonanediol di(meth)acrylate, and neopentylglycol di(meth)acrylate. These may be used singly or in combination of two or more thereof. These may be used singly or in combination of two or more thereof.

### [Other monomer]

The amide group-containing polymer may contain a repeating unit derived from other monomer than aforementioned monomers.

Specific examples of other monomers include acrylonitrile, an alkoxy polyalkylene glycol (meth)acrylate, dimethylaminoethyl methacrylate, vinyl acetate, a vinyl alkyl ether, etc. Other monomers are not limited to these examples. These may be used singly or in combination of two or more thereof.

### [Composition of amide group-containing polymer, etc.]

The amount of repeating units derived from amide group-containing monomer may be 5% by weight or more, 15% by weight or more, 20% by weight or more, 25% by weight or more, 35% by weight or more, 45% by weight or more, 55% by weight or more, or 65% by weight or more, relative to the amide group-containing polymer. The amount of repeating units derived from amide group-containing monomer may be 98% by weight or less, 95% by weight or less, 90% by weight or less, 80% by weight or less, 70% by weight or less, or 60% by weight or less, relative to the amide group-containing polymer.

The amount of repeating units derived from the hydrocarbon-based monomer may be 5% by weight or more, 15% by weight or more, 20% by weight or more, 25% by weight or more, 35% by weight or more, 45% by weight or more, 55% by weight or more, or 65% by weight or more, relative to the amide group-containing polymer. The amount of repeating units derived from the hydrocarbon-based monomer may be 98% by weight or less, 95% by weight or less, 90% by weight or less, 80% by weight or less, 70% by weight or less, or 60% by weight or less, relative to the amide group-containing polymer. Among the hydrocarbon-based monomers, the cyclic hydrocarbon group-containing monomer may be 1% by weight or more, 3% by weight or more, 5% by weight or more, 10% by weight or more, 20% by weight or more, 30% by weight or more, 50% by weight or more, or 75% by weight or more. Among the hydrocarbon-based monomers, the cyclic hydrocarbon group-containing monomer may be 80% by weight or less, 60% by weight or less, 40% by weight or less, or 20% by weight or less.

The amount of repeating units derived from halogenated olefin monomer may be 3% by weight or more, 5% by weight or more, 10% by weight or more, 15% by weight or more, 20% by weight or more, 25% by weight or more, or 35% by weight or more, relative to the amide group-containing polymer. The amount of repeating units derived from halogenated olefin monomer may be 80% by weight or less, 70% by weight or less, 60% by weight or less, 50% by weight or less, 40% by weight or less, 30% by weight or less, 20% by weight or less, or 10% by weight or less, relative to the amide group-containing polymer and preferably 60% by weight or less.

The amount of repeating units derived from cyclic hydrocarbon group-containing monomer may be 0.5% by weight or more, 1% by weight or more, 3% by weight or more, or 4% by weight or more, relative to the amide group-containing polymer. The amount of repeating units derived from halogenated olefin monomer may be 30% by weight or less, 20% by weight or less, 15% by weight or less, 10% by weight or less, 7.5% by weight or less, or 5% by weight or less, relative to the amide group-containing polymer.

The amount of repeating units derived from crosslinkable monomer may be 0.5% by weight or more, 1% by weight or more, 3% by weight or more, 5% by weight or more, 10% by weight or more, or 20% by weight or more, relative to the amide group-containing polymer. The amount of repeating units derived from crosslinkable monomer may be 70% by weight or less, 60% by weight or less, 50% by weight or less, 40% by weight or less, 30% by weight or less, or 20% by weight or less, relative to the amide group-containing polymer.

The weight average molecular weight of the amide group-containing polymer may be 500 or more, 1,000 or more, 2,500 or more, 5,000 or more, 10,000 or more, 25,000 or more, or 50,000 or more, and is preferably 5,000 or more. The weight average molecular weight of the amide group-containing polymer may be 1,000,000 or less, 500,000 or less, 250,000 or less, 100,000 or less, 50,000 or less, 25,000 or less, or 10,000 or less, and is preferably 100,000 or less.

The amount of repeating units derived from other monomer may be 0.5% by weight or more, 1% by weight or more, 3% by weight or more, 5% by weight or more, 10% by weight or less, or 20% by weight or less, relative to the amide group-containing polymer. The amount of repeating units derived from other monomer may be 70% by weight or less, 60% by weight or less, 50% by weight or less, 40% by weight or less, 30% by weight or less, or 20% by weight or less, relative to the amide group-containing polymer.

### <Method for producing amide group-containing polymer>

The method for producing an amide group-containing polymer in the present disclosure includes polymerizing the above-described amide group-containing monomer. In the polymerization, a monomer other than the amide group-containing monomer, for example, at least one selected from the group consisting of a hydrocarbon-based monomer, a crosslinkable monomer, and a halogenated olefin, may be further polymerized. A monomer charge ratio can be changed according to a desired composition of the amide group-containing polymer, and may be the same as the monomer charge ratio of the desired composition of the amide group-containing polymer.

The amide group-containing polymer can be produced by any of ordinary polymerization methods, and the conditions of the polymerization reaction can be arbitrarily selected. Examples of such a polymerization method include solution polymerization, suspension polymerization, and emulsion polymerization.

In solution polymerization, a method is employed that includes dissolving a monomer in an organic solvent in the presence of a polymerization initiator followed by substitution with nitrogen, and then heating and stirring the mixture in a range of 0 to 180°C, for example, 30 to 120°C for 1 to 10 hours. Examples of the polymerization initiator include azobisisobutyronitrile, benzoyl peroxide, di-t-butyl peroxide, lauryl peroxide, cumene hydroperoxide, t-butyl peroxypivalate, and diisopropyl peroxydicarbonate. The polymerization initiator is used in an amount within a range of 0.01 to 20 parts by weight, for example, 0.01 to 10 parts by weight, per 100 parts by weight of the monomer.

The organic solvent is a solvent that is inert to the monomer and dissolves them, and it may be esters (for example, esters having 2 to 40 carbon atoms, specifically ethyl acetate and butyl acetate), ketones (for example, ketones having 2 to 40 carbon atoms, specifically methyl ethyl ketone, diisobutyl ketone, and methyl isobutyl ketone), alcohols (for example, alcohols having 1 to 40 carbon atoms, specifically ethanol, butanol, and isopropyl alcohol). Specific examples of the organic solvent include acetone, chloroform, HCHC225, isopropyl alcohol, cyclohexane, benzene, toluene, xylene, petroleum ether, tetrahydrofuran, 1,4-dioxane, methyl ethyl ketone, methyl isobutyl ketone, diisobutyl ketone, ethyl acetate, butyl acetate, 1,1,2,2-tetrachloroethane, 1,1,1-trichloroethane, trichloroethylene, perchloroethylene, tetrachlorodifluoroethane, and trichlorotrifluoroethane. The organic solvent is used in an amount within a range of 10 to 3,000 parts by weight, for example, 50 to 2,000 parts by weight, per 100 parts by weight of the total amount of the monomers.

In the emulsion polymerization, a method is employed that includes emulsifying a monomer in water in the presence of a polymerization initiator and an emulsifier followed by substitution with nitrogen, and then carrying out polymerization under stirring at a temperature in a range of 50 to 80°C for 1 to 20 hours. The polymerization initiator used is water-soluble compounds such as 1-hydroxycyclohexylhydroperoxide, 3-carboxypropionyl peroxide, acetyl peroxide, azobisisobutylamidine-dihydrochloride, sodium peroxide, potassium persulfate, and ammonium persulfate, and oil-soluble compounds such as azobisisobutyronitrile, benzoyl peroxide, di-t-butyl peroxide, lauryl peroxide, cumene hydroperoxide, t-butyl peroxypivalate, and diisopropyl peroxydicarbonate. The polymerization initiator is used in an amount within a range of 0.01 to 10 parts by weight per 100 parts by weight of the monomer.

In order to obtain a polymer dispersion with excellent standing stability, it is desirable to micronize a monomer and polymerize it in water by using an emulsification apparatus that can impart powerful pulverization energy, such as a high-pressure homogenizer or ultrasonic homogenizer. As an emulsifier, various types of an anionic, cationic or nonionic emulsifier can also be used, and it is used in an amount within a range of 0.5 to 20 parts by weight per 100 parts by weight of monomer. An anionic and/or nonionic and/or cationic emulsifiers are preferably used. In a case in which the monomers are not completely compatible with each other, a compatibilizer that sufficiently compatibilizes these monomers, such as a water-soluble organic solvent or a low molecular weight monomer, is preferably added. The addition of the compatibilizer can improve emulsifiability and copolymerizability.

The polymerization medium (liquid medium) may be water, an organic solvent, or a mixed solvent of water and an organic solvent. Examples of the polymerization medium may include water, esters (for example, esters having 2 to 30 carbon atoms, specifically ethyl acetate and butyl acetate), ketones (for example, ketones having 2 to 30 carbon atoms, specifically methyl ethyl ketone and diisobutyl ketone), alcohols (for example, alcohols having 1 to 30 carbon atoms, specifically isopropyl alcohol), ethers, alkanes, toluene-based solvents, halogenated carbon, etc. Specific examples of other solvents include acetone, isopropyl alcohol, chloroform, HCHC225, pentane, hexane, heptane, octane, cyclohexane, benzene, toluene, xylene, petroleum ether, tetrahydrofuran, 1,4-dioxane, methyl ethyl ketone, methyl isobutyl ketone, diisobutyl ketone, ethyl acetate, butyl acetate, 1,1,2,2-tetrachloroethane, 1,1,1-trichloroethane, trichloroethylene, perchloroethylene, tetrachlorodifluoroethane, trichlorotrifluoroethane, mixed solvents thereof, etc. They may be used singly or in combinations of two or more thereof.

The polymerization medium may contain a water-soluble organic solvent. Examples of the water-soluble organic solvents include, for example, acetone, methyl ethyl ketone, ethyl acetate, propylene glycol, dipropylene glycol monomethyl ether, dipropylene glycol, tripropylene glycol, ethanol, and the like, and each may be used in an amount within a range of 1 to 50 parts by weight, for example, 10 to 40 parts by weight, per 100 parts by weight of water. Examples of the low molecular weight monomer also include, for example, methyl methacrylate, glycidyl methacrylate, 2,2,2-trifluoroethyl methacrylate, and the like, and each may be used in an amount within a range of 1 to 50 parts by weight, for example, 10 to 40 parts by weight, per 100 parts by weight of the total amount of the monomers.

A chain transfer agent may be used in the polymerization. The molecular weight of the polymer can be changed according to the amount of the chain transfer agent used. Examples of the chain transfer agent include mercaptan group-containing compounds such as lauryl mercaptan, thioglycol, and thioglycerol (in particular alkyl mercaptan (for example, having 1 to 40 carbon atoms)), and inorganic salts such as sodium hypophosphite and sodium hydrogen sulfite. The amount of the chain transfer agent used may be in the range of 0.01 to 10 part by weight, for example, 0.1 to 5 parts by weight, relative to 100 parts by weight of the total amount of the monomers.

### <Dispersion, water-repellent composition, or oil-resistant agent composition>

The dispersion (preferably a water dispersion) in the present disclosure contains at least an amide group-containing polymer obtained by polymerizing the above-described amide group-containing monomer. It may further contain at least one of the components that will be described below. The dispersion in the present disclosure can be used as a water-repellent composition or an oil-resistant agent composition, and specific examples of their applications include an external treatment agent (surface-treating agent) or an internal treatment agent, a repellent agent (water-repellent agent, oil-repellent agent, or water- and oil-repellent agent), soil resistant agent, soil release agent, release agent, mold release agent (external mold release agent or internal mold release agent), etc.

### [Amide group-containing polymer]

The amount of the amide group-containing polymer may be 0.01% by weight or more, 0.5% by weight or more, 1% by weight or more, 3% by weight or more, 5% by weight or more, 10% by weight or more, 20% by weight or more, or 30% by weight or more, relative to the dispersion. The amount of the amide group-containing polymer may be 60% by weight or less, 50% by weight or less, 40% by weight or less, 30% by weight or less, 20% by weight or less, 10% by weight or less, 5% by weight or less, or 3% by weight or less, relative to the dispersion. For example, the dispersion may be stored at high concentration upon storage, and when used as a repellent agent, it may be added with a liquid medium, if necessary, diluted to an arbitrary concentration, and then used. The dispersion in the present disclosure has improved product stability, thereby making it possible to supply a high-concentration product that has previously been problematic in terms of stability.

### [Hydrocarbon-based polyurethane]

The dispersion may contain a hydrocarbon-based polyurethane. It may be polyurethane having a hydrocarbon group having 5 to 40 carbon atoms. The number of carbon atoms in the hydrocarbon group having 5 to 40 carbon atoms may be 6 or more, 8 or more, 10 or more, 12 or more, 14 or more, 16 or more, or 18 or more, and is preferably 10 or more and more preferably 12 or more. The number of carbon atoms in the hydrocarbon group having 5 to 40 carbon atoms may be 40 or less, 35 or less, 30 or less, 25 or less, 20 or less, 15 or less, or 10 or less, and is preferably 30 or less and more preferably 25 or less.

The hydrocarbon-based polyurethane having a hydrocarbon group having 5 to 40 carbon atoms can be produced by reacting an isocyanate group-containing compound (for example, a monoisocyanate or polyisocyanate, specifically, a diisocyanate) and an active hydrogen-containing compound having a hydrocarbon group having 5 to 40 carbon atoms. The reaction can be carried out, for example, at 80°C for 1 hour or longer.

The isocyanate group-containing compound is not limited, but for example, an aliphatic polyisocyanate compound, alicyclic polyisocyanate compound, aromatic polyisocyanate compound, aromatic-aliphatic polyisocyanate compound, or modified products of these isocyanate compounds, can be used. These may also be used in combinations of two or more thereof. As the isocyanate group-containing compound, the aliphatic polyisocyanate compound, aromatic polyisocyanate compound, or modified products of these isocyanate compounds, are preferred. The isocyanate group-containing compound is not limited, and examples thereof include, for example, an aliphatic polyisocyanate, alicyclic polyisocyanate, aromatic polyisocyanate, and modified polyisocyanates such as dimers and trimers of these compounds. Commercially available products such as "DESMODUR N-100" (trade name, manufactured by Bayer AG), "DURANATE THA-100" (trade name, manufactured by Asahi Kasei Corporation), "DURANATE 24A-100" (trade name, manufactured by Asahi Kasei Corporation), and the like, can be used.

As the aliphatic polyisocyanate compound, tetramethylene diisocyanate, dodecamethylene diisocyanate, hexamethylene diisocyanate (HDI), 2,2,4-trimethylhexamethylene diisocyanate, 2,4,4-trimethylhexamethylene diisocyanate, lysine diisocyanate, 2-methylpentane-1,5-diisocyanate, 3-methylpentane-1,5-diisocyanate, and the like, can be used. These may be used singly or in combinations of two or more thereof.

As the alicyclic polyisocyanate compound, isophorone diisocyanate, hydrogenated xylylene diisocyanate, 4,4'-dicyclohexylmethane diisocyanate, 1,4-cyclohexane diisocyanate, methylcyclohexylene diisocyanate, 1,3-bis(isocyanatemethyl) cyclohexane, and the like, can be used. These may be used singly or in combinations of two or more thereof.

As the aromatic polyisocyanate compound, dialkyldiphenylmethane diisocyanate, tolylene diisocyanate, 2,2'-diphenylmethane diisocyanate, 2,4'-diphenylmethane diisocyanate, 4,4'-diphenylmethane diisocyanate (monomeric MDI), polymethylene polyphenyl polyisocyanate (polymeric MDI), 4,4'-dibenzyl diisocyanate, 1,5-naphthylene diisocyanate, 1,3-phenylene diisocyanate, 1,4-phenylene diisocyanate, and the like, can be used. These may be used singly or in combinations of two or more thereof.

As the aromatic-aliphatic polyisocyanate compound, xylylene diisocyanate, a tetraalkyldiphenylmethane diisocyanate, α, α, α, α-tetramethylxylylene diisocyanate, and the like, can be used. These may be used singly or in combinations of two or more thereof.

As the modified product of the polyisocyanate compound, an isocyanurate modified product, biuret modified product, adduct modified product, carbodiimide modified product, bifunctionally modified product, and the like, can be used. These may be used singly or in combinations of two or more thereof.

Examples of the active hydrogen-containing compound having a hydrocarbon group having 5 to 40 carbon atoms include active hydrogen-containing compounds (monohydric alcohol derivatives or monovalent carboxylic acid derivatives) such as a hydrocarbon group-containing monoalcohol and a hydrocarbon group-containing monocarboxylic acid; active hydrogen-containing compounds (polyhydric alcohol derivatives or polyhydric carboxylic acid derivatives) in which a hydrocarbon group having 5 to 40 carbon atoms was introduced into polyhydric alcohols or polyhydric carboxylic acids such as sorbitan, citrate, and pentaerythritol. The active hydrogen-containing compound having a hydrocarbon group having 5 to 40 carbon atoms has at least one (for example, one, two, or three) active hydrogen.

Preferred example of the active hydrogen-containing compound having a hydrocarbon group having 5 to 40 carbon atoms are the compounds of polyhydric alcohol derivatives or polyhydric carboxylic acid derivatives of sorbitan (1a), citrate (1b), and pentaerythritol (1c), represented by the following formula: 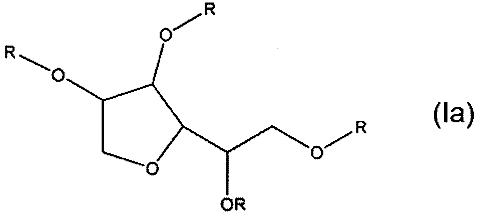 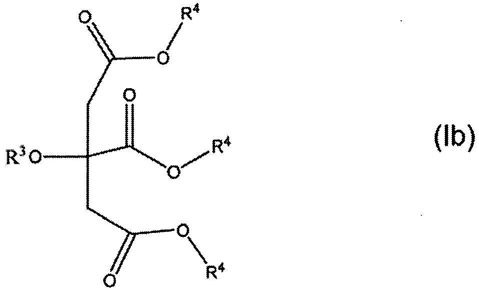 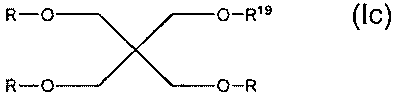 wherein R is each independently -H, -R¹, -C(O)R¹, - (CH₂CH₂O)ₙ(CH(CH₃)CH₂O)ₘR², or -(CH₂CH₂O)ₙ(CH(CH₃)CH₂O)ₘC(O)R¹,
n is each independently 0 to 20,
m is each independently 0 to 20,
m + n is more than 0,
R¹ is each independently a hydrocarbon group having 5 to 40 carbon atoms, optionally including at least one unsaturated bond,
R² is each independently -H or a hydrocarbon group having 5 to 40 carbon atoms, optionally including at least one unsaturated bond,
R³ is each independently -H, -R¹, -C(O)R¹, - (CH₂CH₂O)_{n'}(CH(CH₃)CH₂O)_{m'}R², or - (CH₂CH₂O)_{n'}(CH(CH₃)CH₂O)_{m'}C(O)R¹,
R⁴ is each independently -H, a hydrocarbon group having 5 to 40 carbon atoms, optionally including at least one unsaturated bond, or a combination thereof; - (CH₂CH₂O)_{n'}(CH(CH₃)CH₂O)_{m'}R²; or - (CH₂CH₂O)_{n'}(CH(CH₃)CH₂O)_{m'}C(O)R¹;
n' is each independently 0 to 20,
m' is each independently 0 to 20,
m' + n' is more than 0, and
each R¹⁹ is -H, -C(O)R¹, or -CH₂C[CH₂OR]₃.

When the compound is represented by Formula (Ia), the condition of at least one R or R² is -H,
when the compound is represented by Formula (Ib), the condition of at least one R², R³ or R⁴ is -H, and
when the compound is represented by Formula (Ic), the condition of at least one R¹⁹ or R is -H.

Specific examples of the active hydrogen-containing compound having a hydrocarbon group having 5 to 40 carbon atoms include a sorbitan monocarboxylate, sorbitan dicarboxylate, sorbitan tricarboxylate, monoalkyl citrate, dialkyl citrate, trialkyl citrate, pentaerythritol monocarboxylate, pentaerythritol dicarboxylate, and pentaerythritol tricarboxylate. The carboxylate is preferably stearate or behenate. The hydrocarbon group is preferably an alkyl, and preferred examples of the alkyl are stearyl or behenyl.

The hydrocarbon-based polyurethane may be a dendritic polymer compound (dendrimer) with a radially and regularly branched structure from the center of the compound. The polyurethane obtained by the aforementioned reaction can be a dendritic polymer compound (dendrimer).

For the hydrocarbon-based polyurethane, the descriptions of WO2014/160906, WO2016/049278, and the like, can be referred to.

The amount of hydrocarbon-based polyurethane is 0.01% by weight or more, 0.5% by weight or more, 1% by weight or more, 3% by weight or more, 5% by weight or more, 10% by weight or more, 20% by weight or more, or 30% by weight or more based on the dispersion. The amount of hydrocarbon-based polyurethane is 60% by weight or less, 50% by weight or less, 40% by weight or less, 30% by weight or less, 20% by weight or less, 10% by weight or less, 5% by weight or less, or 3% by weight or less based on the dispersion.

### [Other hydrocarbon-based water-repellent resin]

The hydrocarbon-based water-repellent resin that the dispersion in the present disclosure contains is not limited to the amide group-containing polymer or hydrocarbon-based polyurethane. In place of, or in addition to the hydrocarbon-based acrylic polymer or hydrocarbon-based polyurethane, other hydrocarbon-based water-repellent resin can be used. Examples of the other hydrocarbon-based water-repellent resin include a dendrimer-based resin having a hydrocarbon group at its terminal. Examples of the dendrimer-based resin include RUCO-DRY series (for example, RUCO-DRY DHE, RUCO-DRY ECO, and RUCO-DRY ECO PLUS), manufactured by Rudolph GmbH, and the like.

### [Silicone]

The dispersion in the present disclosure may contain silicone in addition to the amide group-containing polymer. Containing silicone favorably enables combining the water-repellency, oil resistance, and storage stability.

The silicone may be the polymer represented by formula:

(R⁵³)₃Si-O-[-Si(R⁵¹)₂-O-]ₐ-[-Si(R⁵¹)₂-O-]_{b}-Si(R⁵³)₃ (S1)

wherein R⁵¹ each independently represents a hydrogen atom, an alkyl group having 1 to 40 carbon atoms, an aryl group having 6 to 40 carbon atoms, or an alkoxy group having 1 to 40 carbon atoms,
R⁵³ each independently represents a hydrogen atom, an alkyl group having 1 to 40 carbon atoms, an aryl group having 6 to 40 carbon atoms, an alkoxy group having 1 to 40 carbon atoms, or a saturated hydrocarbon group having 1 to 40 carbon atoms,
a represents an integer of 0 or more, b represents an integer of 1 or more, and (a + b) is 5 to 200.

In R⁵¹ and R⁵³, the alkyl group having 1 to 40 carbon atoms and the aryl group having 6 to 40 carbon atoms may be unsubstituted or substituted.

Specific examples of R⁵¹ and R⁵³ include a methyl group, ethyl group, propyl group, butyl group, pentyl group, hexyl group, heptyl group, octyl group, nonyl group, decyl group, dodecyl group, tetradecyl group, hexadecyl group, octadecyl group; cyclopentyl group, cyclohexyl group, cycloheptyl group; phenyl group, tolyl group, naphthyl group, or groups in which hydrogen atoms bonded to these groups are partially or totally substituted with halogen atoms, amino groups, cyano groups, or the like. R⁵¹ and R⁵³ are each preferably a methyl group or ethyl group.

In R⁵¹ and R⁵³, the alkoxy group having 1 to 40 carbon atoms may be linear or branched. Examples of the alkoxy group having 1 to 40 carbon atoms are a methoxy group, ethoxy group, propoxy group, and butoxy group.

The silicone may have at least one long chain hydrocarbon group. For example, at least one of R⁵¹, at least one of R⁵³, or at least one of each of R⁵¹ and R⁵³, in Formula (S1) may be a long chain hydrocarbon group, and at least one (for example, one) of R⁵¹ may be a long chain hydrocarbon group. Herein, the long chain hydrocarbon group may be a saturated hydrocarbon group having 6 or more, 10 or more, 15 or more, or 20 or more carbon atoms, preferably 10 or more or 23 or more carbon atoms. Herein, the hydrocarbon group may be linear or branched, and is preferably an alkyl group. Specific examples of the hydrocarbon group include a hexyl group (6 carbon atoms), octyl group (8 carbon atoms), lauryl group (12 carbon atoms), myristyl group (14 carbon atoms), stearyl group (18 carbon atoms), behenyl group (22 carbon atoms), tricosyl group (23 carbon atoms), lignoceryl group (tetracosyl group, 24 carbon atoms), serotyl group (hexacosyl group, 26 carbon atoms), montyl group (octacosyl group, 28 carbon atoms), melicyl group (triacontane group, 30 carbon atoms), and dotriacontane group (32 carbon atoms).

In terms of ease of industrial manufacture and availability, R⁵¹ and R⁵³ other than the R⁵¹ and R⁵³ that are long chain hydrocarbon groups, are preferably hydrogen atoms or methyl groups and more preferably methyl groups.

a is an integer 0 or more. In terms of ease of industrial manufacture and availability, a may be 40 or less, 30 or less, or 20 or less, and is preferably 30 or less.

The total of a and b is 5 to 200. In terms of ease of industrial manufacture, availability, and handleability, the total of a and b is preferably 10 to 100 and more preferably 40 to 60. a may be 0 to 150, for example, 1 to 100. The lower limit of b may be 1 or 2 or 3, and the upper limit of b may be 150, 10 or 5.

When a or b is 2 or more, each of R⁵¹ and R⁵² that are present in plurality may be the same or different.

50 mol% or more of the total amount of R⁵¹ and R⁵³ groups (for example, the R⁵¹, R⁵² group, and R⁵³ group when represented in the following Formula (S2)) is preferably a methyl group.

The occurrence order of the repeating unit bracketed by a or b is not limited to the occurrence order of the repeating unit represented in the chemical formula, and is arbitrary. Namely, the silicone may be a random polymer or a blocked polymer.

For example, the silicone may be the polymer represented by formula:

(R⁵³)₃Si-O-[-Si(R⁵¹)₂-O-]ₐ-[-Si(R⁵¹)(R⁵²)-O-]_{b}-Si(R⁵³)₃ (S2)

wherein R⁵¹ each independently represents a hydrogen atom, an alkyl group having 1 to 40 carbon atoms, an aryl group having 6 to 40 carbon atoms, an alkoxy group having 1 to 40 carbon atoms, or a long chain hydrocarbon group,
R⁵² each independently represents a long chain hydrocarbon group,
R⁵³ each independently represents a hydrogen atom, an alkyl group having 1 to 40 carbon atoms, an aryl group having 6 to 40 carbon atoms, an alkoxy group having 1 to 40 carbon atoms, or a long chain hydrocarbon group,
a represents an integer of 0 or more, b represents an integer of 1 or more, and (a + b) is 5 to 200.

In Formula (S2), R⁵¹ and R⁵³ may have an alkyl group having 3 to 40 carbon atoms or an unsaturated hydrocarbon group having 6 to 40 carbon atoms (for example, a hydrocarbon group having an aromatic ring), but are preferably free of these groups.

An example of the silicone is as follows: 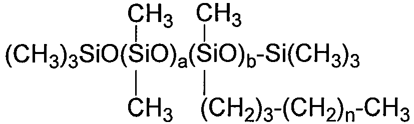 wherein a represents an integer of 0 to 150,
b represents an integer of 1 to 150,
(a + b) is 5 to 200, and
n is an integer of 1 to 36 (n is preferably a long chain hydrocarbon group).

The silicone can be synthesized by conventionally known methods. The silicone can be obtained, for example, by subjecting silicone having a SiH group to a hydrosilylation reaction with an α-olefin.

Examples of the silicone having a SiH group include, for example, methyl hydrogen silicone with a degree of polymerization of 10 to 200, or a copolymer of dimethyl siloxane and methyl hydrogen siloxane. Among them, the methyl hydrogen silicone is preferred in terms of ease of industrial manufacture and availability. The hydrogen silicone (for example, methyl hydrogen silicone) is that in which a side chain of a polydiorganosiloxane is partially substituted with hydrogen and the hydrogen atom is directly connected to the silicon atom. In using the hydrogen silicone, a catalyst may be used to improve its reactivity. For example, zinc, tin, manganese, cobalt, iron, and amine-type catalyst can be used. As these catalysts, an organic acid metal salt is preferred, and as the organic acid, a fatty acid is preferred. From a safety point of view, zinc stearate and the like can be used. Use of 10 to 40% of the catalyst relative to methyl hydrogen silicone, facilitates demonstrating its effect, which is preferred. Two or more types of amino-modified or epoxy-modified silicone, carboxy-modified silicone, and methyl hydrogen silicone may be mixed. Preferably, all of the silicones have reactive groups and are those having film formability. The film formability means that after the silicone in an emulsion state is adhered to a fiber surface, the silicone forms a solid film and does not become an oil or gel.

The α-olefin is a compound from which a long chain hydrocarbon group is derived in silicone. Specific examples of the α-olefin are 1-tricosene, 1-tetracosene, 1-hexacosene, 1-octacosene, 1-triacontene, and 1-dotriacontene.

The hydrosilylation reaction may be carried out by reacting the silicone having a SiH group with the α-olefin stepwisely or all at once in the presence of a catalyst, if necessary.

The amounts of the silicone having a SiH group and α-olefin used in the hydrosilylation reaction can be appropriately selected depending on a SiH group equivalent, the number average molecular weight, or the like, of the silicone having a SiH group.

Examples of the catalyst used in the hydrosilylation reaction include compounds such as platinum and palladium, and among them, the platinum compound being preferred. Examples of the platinum compound include platinum (IV) chloride.

The reaction conditions for the hydrosilylation reaction are not limited and can be appropriately adjusted. A reaction temperature is, for example, 10 to 200°C and preferably 50 to 150°C. A reaction time can be, for example, 3 to 12 hours when the reaction temperature is 50 to 150°C.

The hydrosilylation reaction is preferably carried out under an inert gas atmosphere. Examples of the inert gas include, for example, nitrogen, argon, and the like. The reaction proceeds in the absence of solvent, however, a solvent may also be used. Examples of the solvent include, for example, dioxane, methyl isobutyl ketone, toluene, xylene, butyl acetate, and the like.

### (Reactive Silicone)

The silicone may include reactive silicone. Examples of the reactive silicone include polysiloxanes having a reactive group on a side chain, at one terminal, at each of both terminals, or on a side chain and each of both terminals, and may be a polysiloxane having a reactive group on a side chain and/or at each of both terminals in view of excellent sliding resistance as well as water-repellency at the same time. The reactive silicone is not limited as long as it has a reactive group in its molecule, and examples thereof include, for example, amino-modified silicone, epoxy-modified silicone, carboxy-modified silicone, hydrogen-modified silicone, and the like. The reactive silicone may be silicone in which one or more substituents in the above Formula (S1) or Formula (S2) are substituted with reactive groups.

Examples of the amino-modified silicone include those having a structure in which an amino group is bonded to an organic group directly connected to a silicon atom. The organic group may be either an alkylene group or a divalent aromatic group. The alkylene group is preferably those having 2 or more carbon atoms. The divalent aromatic group is preferably those having 6 or more carbon atoms. The amino group may be any of a primary amino group, secondary amino group, or tertiary amino group. Examples of the organic group to which an amino group is bonded include: a 2-aminoethyl group, N-methyl-2-aminoethyl group, N,N-dimethyl-2-aminoethyl group, N-ethyl-2-aminoethyl group, N,N-diethyl-2-aminoethyl group, N,N-methylethyl-2-aminoethyl group, 3-aminopropyl group, N-methyl-3-aminopropyl group, N,N-dimethyl-3-aminopropyl group, N-ethyl-3-anopropyl group, N,N-diethyl-3-aminopropyl group, and N,N-methylethyl-3-aminopropyl group. These functional groups may be on a side chain of a polysiloxane or at the terminal thereof.

Examples of the epoxy-modified silicone include those having a structure in which an epoxy group is bonded to an organic group directly connected to a silicon atom. The organic group may be either an alkylene group or a divalent aromatic group. The epoxy group in this case usually bonds to the organic group to form a glycidyl ether. Examples of such a functional group include a 3-glycidoxypropyl group and 2-glycidoxyethyl group. These functional groups may be on a side chain of a polysiloxane or at the terminal thereof.

Examples of the carboxy-modified silicones include those having a structure in which a carboxy group is bonded to an organic group directly connected to a silicon atom. The organic group may be either an alkylene group or a divalent aromatic group. The alkylene group is preferably those having 2 or more carbon atoms. The divalent aromatic group is preferably those having 6 or more carbon atoms. Examples of such a functional group include a 3-carboxypropyl group and 2-carboxyethyl group. These functional groups may be on a side chain of a polysiloxane or at the terminal thereof.

### (Amount of Silicone)

The amount of silicone may be 0.1 parts by weight or more, 1 part by weight or more, 3 parts by weight or more, 5 parts by weight or more, 10 parts by weight or more, 15 parts by weight or more, or 20 parts by weight or more, per 100 parts by weight of the amide group-containing polymer. The amount of silicone is 50 parts by weight or less, 40 parts by weight or less, 30 parts by weight or less, 20 parts by weight or less, 10 parts by weight or less, or 5 parts by weight or less, per 100 parts by weight of the amide group-containing polymer.

### [Wax]

The dispersion in the present disclosure may contain wax in addition to the amide group-containing polymer. Containing the wax can favorably combine the water-repellency, oil resistance, and storage stability. The dispersion in the present disclosure may contain both silicone and wax, or may contain either silicone or wax singly.

Examples of the wax include paraffin wax, microcrystalline wax, Fischer-Tropsch wax, polyolefin wax (polyethylene wax, polypropylene wax, etc.), oxidized polyolefin wax, animal and vegetable wax, mineral wax, and the like. The paraffin wax is preferred. Specific examples of compounds constituting the wax include normal alkanes (for example, tricosane, tetracosane, pentacosane, hexacosane, heptacosane, octacosane, nonacosane, triacontane, hentriacontane, dotriacontane, tritriacontane, tetratriacontane, pentatriacontane, and hexatriacontane), normal alkenes (for example, 1-eicosene, 1-docosene, 1-tricosene, 1-tetracosene, 1-pentacosene, 1-hexacosene, 1-heptacosene, 1-octacosene, nonacosane, triacontane, hentriacontane, dotriacontane, tritriacontane, tetratriacontane, pentatriacontane, and hexatriacontane). The number of carbon atoms in the compounds constituting the wax is preferably 20 to 60, for example 25 to 45. The molecular weight of the wax may be 200 to 2,000, for example, 250 to 1,500, or 300 to 1,000. These may be used singly or in combinations of two or more thereof.

The melting point of the wax may be 50°C or higher, 55°C or higher, 60°C or higher, 65°C or higher, or 70°C or higher, and is preferably 55°C or higher and more preferably 60°C or higher. The melting point of the wax is measured in accordance with JIS K 2235-1991.

### (Amount of Wax)

The amount of wax may be 0.1 parts by weight or more, 1 part by weight or more, 3 parts by weight or more, 5 parts by weight or more, 10 parts by weight or more, 15 parts by weight or more, or 20 parts by weight or more, per 100 parts by weight of the amide group-containing polymer. The amount of wax may be 50 parts by weight or less, 40 parts by weight or less, 30 parts by weight or less, 20 parts by weight or less, 10 parts by weight or less, or 5 parts by weight or less, per 100 parts by weight of the amide group-containing polymer.

### [Liquid medium]

The dispersion contains a liquid medium. The dispersion is preferably a water dispersion containing water as the liquid medium. The liquid medium is water, an organic solvent, or a mixture of water and an organic solvent. It is preferably a mixture of water and an organic solvent. Containing the organic solvent can favorably combine the water-repellency, oil resistance, and storage stability.

Examples of the organic solvent include esters (for example, esters having 2 to 40 carbon atoms, specifically ethyl acetate and butyl acetate), ketones (for example, ketones having 2 to 40 carbon atoms, specifically methyl ethyl ketone and diisobutyl ketones), alcohols (for example, alcohols having 1 to 40 carbon atoms, specifically isopropyl alcohol), aromatic solvents (for example, toluene and xylene), petroleum-based solvents (for example, alkanes having 5 to 10 carbon atoms, specifically, naphtha and kerosene). The organic solvent is preferably a water-soluble organic solvent. The water-soluble organic solvent may contain a compound having at least one hydroxy group (for example, an alcohol, a polyhydric alcohol such as a glycol-based solvent, an ether (for example, monoether) of polyhydric alcohol, and the like). These may be used singly or in combinations of two or more thereof.

### (Amount of Liquid Medium)

The amount of liquid medium may be 40% by weight or more, 50% by weight or more, 60% by weight or more, 70% by weight or more, 80% by weight or more, 90% by weight or more, 95% by weight or more, or 97% by weight or more based on the dispersion. The amount of the liquid medium is 99.9% by weight or less, 99% by weight or less, 95% by weight or less, 90% by weight or less, 80% by weight or less, 70% by weight or less, 60% by weight or less, or 50% by weight or less based on the dispersion.

The amount of the organic solvent may be 0.5% by weight or more, 1% by weight or more, 2% by weight or more, 3% by weight or more, 5% by weight or more, 7.5% by weight or more, 10% by weight or more, 12.5% by weight or more, 15% by weight or more, or 20% by weight or more based on the dispersion. The amount of the organic solvent may be 75% by weight or less, 50% by weight or less, 40% by weight or less, 30% by weight or less, 25% by weight or less, 20% by weight or less, 15% by weight or less, 10% by weight or less, or 5% by weight or less based on the dispersion.

The amount of the organic solvent may be 1% by weight or more, 3% by weight or more, 5% by weight or more, 10% by weight or more, 20% by weight or more, 30% by weight or more, or 40% by weight or more based on the liquid medium. The amount of organic solvent may be 55% by weight or less, 45% by weight or less, 35% by weight or less, 25% by weight or less, 15% by weight or less, 12.5% by weight or less, 7.5% by weight or less, or 5.0% by weight or less based on the liquid medium.

The amount of the organic solvent is 1 part by weight or more, 3 parts by weight or more, 5 parts by weight or more, 10 parts by weight or more, 20 parts by weight or more, 30 parts by weight or more, 40 parts by weight or more, or 50 parts by weight or more, per 100 parts by weight of the amide group-containing polymer. The amount of the organic solvent is 200 parts by weight or less, 175 parts by weight or less, 150 parts by weight or less, 125 parts by weight or less, 100 parts by weight or less, 80 parts by weight or less, 60 parts by weight or less, 40 parts by weight or less, 20 parts by weight or less, or 10 parts by weight or less, per 100 parts by weight of the amide group-containing polymer.

The amount of organic solvent may be 0.5 parts by weight or more, 1 part by weight or more, 1.5 parts by weight or more, 3 parts by weight or more, 5 parts by weight or more, 10 parts by weight or more, 15 parts by weight or more, 20 parts by weight or more, 30 parts by weight or more, or 40 parts by weight or more, per 100 parts by weight of water. The amount of organic solvent may be 100 parts by weight or less, 75 parts by weight or less, 50 parts by weight or less, 25 parts by weight or less, 10 parts by weight or less, or 5 parts by weight or less, per 100 parts by weight of water.

### [Organic Acid]

The dispersion may also contain an organic acid. As the organic acid, a known organic acid can be used. Examples of the organic acid preferably include a carboxylic acid, sulfonic acid, sulfinic acid, and the like, with the carboxylic acid being particularly preferred. Examples of the carboxylic acid include formic acid, acetic acid, propionic acid, butyric acid, oxalic acid, succinic acid, glutaric acid, adipic acid, malic acid, citric acid, and the like, with the formic acid or acetic acid being particularly preferred. In the present disclosure, one type of organic acid may be used, or two or more types thereof may be combined for use. For example, formic acid and acetic acid may be combined for use.

### (Amount of Organic Acid)

The amount of organic acid may be 0.1 parts by weight or more, 1 part by weight or more, 3 parts by weight or more, 5 parts by weight or more, 10 parts by weight or more, 15 parts by weight or more, or 20 parts by weight or more, per 100 parts by weight of the amide group-containing polymer. The amount of organic acid may be 50 parts by weight or less, 40 parts by weight or less, 30 parts by weight or less, 20 parts by weight or less, 10 parts by weight or less, or 5 parts by weight or less, per 100 parts by weight of the amide group-containing polymer. The amount of organic acid may be adjusted so that the pH of the dispersion is 3 to 10, for example 5 to 9 and particularly 6 to 8. The dispersion may be acidic (pH 7 or less, for example 6 or less).

### [Surfactant]

The dispersion preferably contains a surfactant. In the dispersion, the surfactant may include a nonionic surfactant. Containing the surfactant can favorably combine the water-repellency, oil resistance, and storage stability. Further, the surfactant may include one or more surfactants selected from a cationic surfactant, an anionic surfactant, and an amphoteric surfactant. A combination of the nonionic surfactant and the cationic surfactant is preferably used.

### (Nonionic Surfactant)

Examples of the nonionic surfactant include an ether, ester, ester ether, alkanolamide, polyhydric alcohol, and amine oxide.

An example of the ether is a compound having an oxyalkylene group (preferably a polyoxyethylene group).

Examples of the ester are an ester of an alcohol and a fatty acid. Examples of the alcohol are a monovalent to hexavalent (particularly divalent to pentavalent) alcohol having 1 to 50 carbon atoms (particularly 10 to 30 carbon atoms) (for example, an aliphatic alcohol). An example of the fatty acid is a saturated or unsaturated fatty acid having 2 to 50 carbon atoms, particularly 5 to 30 carbon atoms.

An example of the ester ether is a compound obtained by adding an alkylene oxide (particularly ethylene oxide) to an ester of an alcohol and a fatty acid. Examples of the alcohol are a monovalent to hexavalent (particularly divalent to pentavalent) alcohol having 1 to 50 carbon atoms (particularly 3 to 30 carbon atoms) (for example, an aliphatic alcohol). An example of the fatty acid is a saturated or unsaturated fatty acid having 2 to 50 carbon atoms, particularly 5 to 30 carbon atoms.

An example of the alkanolamide is a compound formed of a fatty acid and an alkanolamine. The alkanolamide may be a monoalkanolamide or a dialkanolamide. An example of the fatty acid is a saturated or unsaturated fatty acid having 2 to 50 carbon atoms, particularly 5 to 30 carbon atoms. The alkanolamine may be an alkanol having 1 to 3 amino groups and 1 to 5 hydroxyl groups, having 2 to 50 carbon atoms, particularly 5 to 30 carbon atoms.

The polyhydric alcohol may be a divalent to pentavalent alcohol having 10 to 30 carbon atoms.

The amine oxide may be an oxide (for example, having 5 to 50 carbon atoms) of an amine (secondary amine or preferably tertiary amine).

The nonionic surfactant is preferably a nonionic surfactant having an oxyalkylene group (preferably a polyoxyethylene group). The number of carbon atoms of the alkylene group in the oxyalkylene group is preferably 2 to 10. The number of oxyalkylene groups in a molecule of the nonionic surfactant is generally preferably 2 to 100.

The nonionic surfactant is selected from the group consisting of an ether, ester, ester ether, alkanolamide, polyhydric alcohol, and amine oxide, and is preferably a nonionic surfactant having an oxyalkylene group.

The nonionic surfactant may be an alkylene oxide adduct of a linear and/or branched aliphatic (saturated and/or unsaturated) group, a polyalkylene glycol ester of a linear and/or branched fatty acid (saturated and/or unsaturated), a polyoxyethylene (POE)/polyoxypropylene (POP) copolymer (random or block copolymer), an alkylene oxide adduct of acetylene glycol, etc. Among them, the nonionic surfactant is preferably a surfactant such that the structures of the alkylene oxide addition moiety and polyalkylene glycol moiety are polyoxyethylene (POE) or polyoxypropylene (POP) or POE/POP copolymer (which may be a random or block copolymer).

The nonionic surfactant also preferably has a structure free of an aromatic group due to environmental issues (biodegradability, environmental hormones, and the like).

The nonionic surfactant may be the compound represented by formula:

R¹O-(CH₂CH₂O)ₚ-(R²O)_{q}-R³

wherein R¹ is an alkyl group having 1 to 22 carbon atoms, an alkenyl group having 2 to 22 carbon atoms, or an acyl group,
R² is each independently the same or different and is an alkylene group having 3 or more carbon atoms (for example, 3 to 10),
R³ is a hydrogen atom, an alkyl group having 1 to 22 carbon atoms, or an alkenyl group having 2 to 22 carbon atoms,
p is a numeral of 2 or more, and
q is 0 or a numeral of 1 or more.

R¹ preferably has 8 to 20 carbon atoms, particularly 10 to 18 carbon atoms. Preferred specific examples of R¹ include a lauryl group, tridecyl group, and oleyl group.

Examples of R² are a propylene group and butylene group.

In the nonionic surfactant, p may be a numeral of 3 or more (for example, 5 to 200). q may be a numeral of 2 or more (for example, 5 to 200). That is, -(R²O)_{q}- may form a polyoxyalkylene chain.

The nonionic surfactant may be a polyoxyethylene alkylene alkyl ether including a hydrophilic polyoxyethylene chain and a hydrophobic oxyalkylene chain (particularly a polyoxyalkylene chain) in the center. Examples of the hydrophobic oxyalkylene chain include an oxypropylene chain, an oxybutylene chain, a styrene chain, and the like, and among them, the oxypropylene chain is preferred.

Specific examples of the nonionic surfactant include condensation products of ethylene oxide with hexylphenol, isooctylphenol, hexadecanol, oleic acid, an alkane (C₁₂-C₁₆) thiol, a sorbitan monofatty acid (C₇-C₁₉) or alkyl (C₁₂-C₁₈) amine, and the like.

A proportion of a polyoxyethylene block can be 5 to 80% by weight, for example, 30 to 75% by weight, and particularly 40 to 70% by weight based on the molecular weight of the nonionic surfactant (copolymer).

An average molecular weight of the nonionic surfactant is generally 300 to 5,000, for example, 500 to 3,000.

The nonionic surfactant may be a mixture of a compound with an HLB (hydrophilicity - hydrophobicity balance) of less than 15 (particularly 5 or less) and a compound with an HLB of 15 or more. An example of the compound with an HLB of less than 15 is a sorbitan fatty acid ester. An example of the compound with an HLB of 15 or more is a polyoxyethylene alkyl ether. A weight ratio of the compound with an HLB of less than 15 to the compound with an HLB of 15 or more may be 90:10 to 20:80, for example, 85:15 to 55:45.

The nonionic surfactant may be used singly or in admixture of two or more thereof.

### (Cationic Surfactant)

The cationic surfactant is preferably a compound free of an amide group.

The cationic surfactant may be an amine salt, quaternary ammonium salt, or oxyethylene-added ammonium salt. Specific examples of the cationic surfactant include, but are not limited to, an alkyl amine salt, amine salt type surfactants such as an amino alcohol fatty acid derivative, polyamine fatty acid derivative, and imidazoline, and quaternary ammonium salt type surfactants such as an alkyl trimethylammonium salt, dialkyl dimethylammonium salt, alkyl dimethyl benzyl ammonium salt, pyridinium salt, alkyl isoquinolinium salt, and benzethonium chloride.

Preferred examples of the cationic surfactant are the compounds of:

R²¹-N+(-R²²)(-R²³)(-R²⁴) X⁻

wherein R²¹, R²², R²³ and R²⁴ are hydrocarbon groups having 1 to 40 carbon atoms, and
X is an anionic group.

Specific examples of R²¹, R²², R²³ and -R²⁴ are alkyl groups (for example, a methyl group, butyl group, stearyl group, and palmityl group). Specific examples of X are halogen (for example, chlorine), acids (for example, hydrochloric acid and acetic acid).

The cationic surfactant is particularly preferably a monoalkyltrimethylammonium salt (the number of carbon atoms of the alkyl is 4 to 40).

The cationic surfactant is preferably an ammonium salt. The cationic surfactant may be the ammonium salt represented by formula:

R¹ₚ-N⁺R²_{q}X⁻

wherein R¹ is a C12 or more (for example, C₁₂ to C₅₀) linear and/or branched aliphatic (saturated and/or unsaturated) group,
R² is H or a C1 to C4 alkyl group, benzyl group, polyoxyethylene group (the number of oxyethylene groups is for example, 1 (particularly 2, especially 3) to 50)
(CH₃ and C₂H₅ are particularly preferred),
X is a halogen atom (for example), a C₁ to C₄ fatty acid base,
p is 1 or 2, q is 2 or 3, and p + q = 4. The number of carbon atoms of R¹ may be 12 to 50, for example, 12 to 30.

Specific examples of the cationic surfactant include dodecyltrimethylammonium acetate, trimethyltetradecylammonium chloride, hexadecyltrimethylammonium bromide, trimethyloctadecylammonium chloride, (dodecylmethylbenzyl)trimethylammonium chloride, benzyldodecyldimethylammonium chloride, methyldodecyldi(hydropolyoxyethylene)ammonium chloride, benzyldodecyldi(hydropolyoxyethylene)ammonium chloride, and N-[2-(diethylamino)ethyl]oleamide hydrochloride.

Examples of the anionic surfactant include an alkyl ether sulfate, alkyl sulfate, alkenyl ether sulfate, alkenyl sulfate, olefin sulfonate, alkanesulfonate, saturated or unsaturated fatty acid salt, alkyl or alkenyl ether carbonate, α-sulfone fatty acid salt, N-acylamino acid type surfactant, phosphate mono- or diester-type surfactant, and sulfosuccinic acid ester.

Examples of the amphoteric surfactant includes alanines, imidazolinium betaines, amido betaines, and betaine acetate. Specific examples include lauryl betaine, stearyl betaine, lauryl carboxymethyl hydroxyethyl imidazolinium betaine, lauryl dimethylaminoacetate betaine, and fatty acid amidopropyl dimethylamino acetate betaine.

The surfactant may be one type or a combination of two or more of each of the nonionic surfactant, cationic surfactant, and amphoteric surfactants.

### (Amount of Surfactant)

The amount of the surfactant may be 0.1 parts by weight or more, 1 part by weight or more, 3 parts by weight or more, 5 parts by weight or more, 10 parts by weight or more, 15 parts by weight or more, or 20 parts by weight or more, per 100 parts by weight of the amide group-containing polymer. The amount of the surfactant may be 50 parts by weight or less, 40 parts by weight or less, 30 parts by weight or less, 20 parts by weight or less, 10 parts by weight or less, or 5 parts by weight or less, per 100 parts by weight of the amide group-containing polymer.

The amount of the cationic surfactant may be 5% by weight or more, preferably 10% by weight or more, and more preferably 20% by weight or more based on the total amount of the surfactants. The weight ratio of the nonionic surfactant and the cationic surfactant is preferably 95:5 to 20:80 and more preferably 85:15 to 40:60.

The amount of the cationic surfactant may be 0.05 to 10 parts by weight, for example, 0.1 to 8 parts by weight, per 100 parts by weight of the amide group-containing polymer. The total amount of surfactants may be 0.1 to 20 parts by weight, for example, 0.2 to 10 parts by weight, per 100 parts by weight of the amide group-containing polymer.

### [Curing Agent]

The dispersion may contain a curing agent (active hydrogen-reactive compound or active hydrogen-containing compound). The curing agent may be added to the dispersion after a amide group-containing polymer was obtained by polymerization.

The curing agent (crosslinking agent) in the dispersion can favorably cure the amide group-containing polymer. The curing agent may be an active hydrogen-reactive compound or an active hydrogen-containing compound that reacts with active hydrogen or an active hydrogen-reactive group of the amide group-containing polymer. Examples of the active hydrogen-reactive compound include a polyisocyanate compound, an epoxy compound, a chloromethyl group-containing compound, a carboxyl group-containing compound, and a hydrazide compound. Examples of the active hydrogen-containing compound include a hydroxyl group-containing compound, an amino group-containing compound and a carboxyl group-containing compound, a ketone group-containing compound, a hydrazide compound, and a melamine compound.

The curing agent may be a polyisocyanate compound. The polyisocyanate compound is a compound having two or more isocyanate groups in one molecule. The polyisocyanate compound serves as a crosslinking agent. Examples of the polyisocyanate compound include an aliphatic polyisocyanate, alicyclic polyisocyanate, aromatic-aliphatic polyisocyanate, aromatic polyisocyanate, and derivatives of these polyisocyanates.

Examples of the aliphatic polyisocyanate include aliphatic diisocyanates such as trimethylene diisocyanate, tetramethylene diisocyanate, hexamethylene diisocyanate, pentamethylene diisocyanate, 1,2-propylene diisocyanate, 1,2-butylene diisocyanate, 2,3-butylene diisocyanate 1,3-butylene diisocyanate, 2,4,4- or 2,2,4-trimethylhexamethylene diisocyanate, and 2,6-diisocyanatomethylcaproate, and aliphatic triisocyanates such as lysine ester triisocyanate, 1,4,8-triisocyanatooctane, 1,6,11-triisocyanatoundecane, 1,8-diisocyanato-4-(isocyanatomethyl)octane, 1,3,6-triisocyanatohexane, and 2,5,7-trimethyl-1,8-diisocyanato-5-(isocyanatomethyl)octane. These may be used singly or in combination of two or more thereof.

Examples of the alicyclic polyisocyanate include an alicyclic diisocyanate and alicyclic triisocyanate. Specific examples of the alicyclic polyisocyanate include 1,3-cyclopentene diisocyanate, 3-isocyanatomethyl-3,5,5-trimethylcyclohexyl isocyanate (isophorone diisocyanate), and 1,3, 5-triisocyanatocyclohexane. These may be used singly or in combination of two or more thereof.

Examples of the aromatic-aliphatic polyisocyanate include an aromatic-aliphatic diisocyanate and aromatic-aliphatic triisocyanate. Specific examples of the aromatic-aliphatic polyisocyanate include 1,3- or 1,4-xylylene diisocyanate or a mixture thereof, 1,3- or 1,4-bis(1-isocyanato-1-methylethyl)benzene (tetramethyl xylylene diisocyanate) or a mixture thereof, and 1,3,5-triisocyanatomethylbenzene. These may be used singly or in combination of two or more thereof.

Examples of the aromatic polyisocyanates include an aromatic diisocyanate, aromatic triisocyanate, and aromatic tetraisocyanate. Specific examples of aromatic polyisocyanates include m-phenylenediisocyanate, p-phenylenediisocyanate, 4,4'-diphenyl diisocyanate, 1,5-naphthalene diisocyanate, 2,4'- or 4,4'-diphenylmethane diisocyanate or a mixture thereof, 2,4- or 2,6-tolylenediisocyanate or a mixture thereof, triphenylmethane-4,4',4''-triisocyanate, and 4,4'-diphenylmethane-2,2',5,5'-tetraisocyanate. These may be used singly or in combination of two or more thereof.

Examples of the derivative of the polyisocyanate include various derivatives such as a dimer, trimer, biuret, allophanate, carbodiimide, urethodione, urethoimine, isocyanurate, and iminooxadiazinedione of the aforementioned polyisocyanate compounds. These may be used singly or in combination of two or more thereof.

These polyisocyanates can be used singly or in combination of two or more thereof.

As the polyisocyanate compound, a blocked polyisocyanate compound (blocked isocyanate), which is a compound obtained by blocking isocyanate groups of the polyisocyanate compound with a blocking agent, is preferably used. The blocked polyisocyanate compound is preferably used because it is relatively stable even in an aqueous solution and can be used in the same aqueous solution as the dispersion.

The blocking agent is an agent that blocks free isocyanate groups. The blocked polyisocyanate compound, for example, can be heated 100°C or higher, for example, 130°C or higher to regenerate isocyanate groups, facilitating a reaction with hydroxyl groups. Examples of the blocking agent include a phenol-based compound, lactam-based compound, aliphatic alcohol-based compound, and oxime-based compound. The polyisocyanate compounds can be used singly or in combination of two or more thereof.

The epoxy compound is a compound having an epoxy group. Examples of the epoxy compound include an epoxy compound having a polyoxyalkylene group, for example, a polyglycerol polyglycidyl ether and polypropylene glycol diglycidyl ether; and sorbitol polyglycidyl ether.

The chloromethyl group-containing compound is a compound having a chloromethyl group. Examples of the chloromethyl group-containing compound include chloromethyl polystyrene.

The carboxyl group-containing compound is a compound having a carboxyl group. Examples of the carboxyl group-containing compound include (poly)acrylic acid, and (poly)methacrylic acid.

Specific examples of the ketone group-containing compound include (poly)diacetone acrylamide, and diacetone alcohol.

Specific examples of the hydrazide compound include hydrazine, carbohydrazide, and adipic acid hydrazide.

Specific examples of the melamine compound include a melamine resin, and methyl etherified melamine resin.

### (Amount of Curing Agent)

The amount of the curing agent is 0.1 parts by weight or more, 1 part by weight or more, 3 parts by weight or more, 5 parts by weight or more, 10 parts by weight or more, 15 parts by weight or more, or 20 parts by weight or more, per 100 parts by weight of the amide group-containing polymer. The amount of the curing agent is 50 parts by weight or less, 40 parts by weight or less, 30 parts by weight or less, 20 parts by weight or less, 10 parts by weight or less, or 5 parts by weight or less, per 100 parts by weight of the amide group-containing polymer.

### [Other Component]

The dispersion may contain other component other than the aforementioned components. Examples of other components include water- and/or oil- repellent agents, anti-slip agents, antistatic agents, antiseptic agents, ultraviolet absorbers, antibacterial agents, deodorants, and perfumes. These may be used singly or in combination of two or more thereof. In addition to the above components, examples of other components include texture modifiers, softening agents, antibacterial agents, flame retardants, coating material fixing agents, wrinkle-resistant agents, drying rate adjusters, crosslinking agents, film formation agents, compatibilizing agents, antifreezing agents, viscosity modifiers, ultraviolet absorbers, antioxidants, pH adjusters, insect repellents, antifoaming agents, anti-shrinkage agents, laundry wrinkle-resistant agents, shape retention agents, drape retention agents, ironing improving agents, whitening agents, bleaching agents, fabric softening clays, anti-dye transfer agents such as polyvinylpyrrolidone, polymer dispersants, soil release agents, scum dispersants, fluorescent brightening agents such as 4,4-bis(2-sulfostyryl)biphenyl disodium (Tinopearl CBS-X manufactured by Ciba Specialty Chemicals Corporation), dye fixing agents, anti-color fading agents such as 1,4-bis(3-aminopropyl) piperazine, stain removing agents, enzymes such as cellulase, amylase, protease, lipase, and keratinase as fiber surface modifiers, foam inhibitors, silk protein powder that can impart silky texture and functions such as moisture absorption and desorption properties, surface modifiers thereof, emulsified dispersions, and specifically K-50, K-30, K-10, A-705, S-702, L-710, FP series (Idemitsu Petrochemical Co., Ltd.), hydrolyzed silk liquid (Jomo), Silk Gen G Solble S (ICHIMARU FHARCOS Co., Ltd.), a nonionic polymer compound composed of alkylene terephthalate and/or alkylene isophthalate units and polyoxyalkylene units, for example, antifouling agent such as FR627 manufactured by GOO CHEMICAL CO., LTD. and SRC-1 manufactured by Clariant Japan K. K, can be compounded. These may be used singly or in combination of two or more thereof.

### (Antistatic agent)

Examples of the antistatic agent include cationic antistatic agents with cationic functional groups such as quaternary ammonium salts, pyridinium salts, primary-, secondary-, and tertiary-amino groups; anionic antistatic agents with anionic functional groups such as sulfonic acid salts or sulfuric acid ester salts, phosphonic acid salts, phosphoric acid ester salts; amphoteric antistatic agents such as alkyl betaine and derivatives thereof, imidazoline and derivatives thereof, alanine and derivatives thereof, and nonionic antistatic agents such as amino alcohols and derivatives thereof, glycerin and derivatives thereof, and polyethylene glycol and derivatives thereof. The antistatic agent may be an ion-electric conductive polymer obtained by polymerizing or copolymerizing a monomer having these cationic, anionic or amphoteric ion-electric conductive groups. These may be used singly or in combination of two or more thereof.

### (Antiseptic agent)

The antiseptic agent may be used mainly to enhance antisepsis power and bactericidal power to maintain antiseptic during long-term storage. Examples of the antiseptic agent include isothiazolone-based organosulfur compounds, benzisothiazolone-based organosulfur compounds, benzoic acids, and 2-bromo-2-nitro-1,3-propanediol. The content of antiseptic agent is preferably 0.0001 to 1% by weight relative to the total weight of the dispersion. When the content of antiseptic agent is the lower limit value of the aforementioned range or more, a sufficient effect of antiseptic agent added is obtained, and when the content is the upper limit value or less, favorable storage stability of the dispersion is obtained.

### (Ultraviolet absorber)

The ultraviolet absorber is an agent that has a protection effect against ultraviolet rays, and is a component that absorbs ultraviolet rays, converts them into infrared rays, visible rays, and the like, and emits them. Examples of the ultraviolet absorber include aminobenzoic acid derivatives, salicylic acid derivatives, silicic acid derivatives, benzophenone derivatives, azole-based compounds, and 4-t-butyl-4'-methoxybenzoylmethane.

### (Antibacterial agents)

The antibacterial agent is a component that exhibits the effect of inhibiting bacteria from growing on fibers and further exhibits the effect of inhibiting generation of unpleasant odors derived from decomposition products of microorganisms. Examples of the antibacterial agent include cationic bactericidal agents such as quaternary ammonium salts, zinc bis-(2-pyridylthio-1-oxide), polyhexamethylene biguanidine hydrochloride, 8-oxyquinoline, and polylysine.

### (Deodorants)

Examples of the deodorant include cluster dextrin, methyl-β-cyclodextrin, 2-hydroxypropyl-β-cyclodextrin, monoacetyl-β-cyclodextrin, acylamidopropyl dimethylamine oxide, and an aminocarboxylic acid-based metal complex (the zinc complex of trisodium methylglycine diacetate described in WO2012/090580).

### (Perfume)

The perfumes are not limited, and a list of usable perfume materials can be found in various literatures, including "Perfume and Flavor Chemicals", Vol. I and II, Steffen Arctander, Allured Pub. Co. (1994) and "Synthetic Perfume Chemistry and Product Knowledge", Genichi Indo, The Chemical Daily Co., Ltd. (1996) and "Perfume and Flavor Materials of Natural Origin", Steffen Arctander, Allured Pub. Co. (1994) and "Encyclopedia of Fragrance", edited by the Japan Perfume Association, Asakura Publishing Co., Ltd. (1989) and "Perfumery Material Performance V.3.3", Boelens Aroma Chemical Information Service (1996) and "Flower oils and Floral Compounds In Perfumery", Danute Lajaujis Anonis, Allured Pub. Co. (1993).

### (Amount of other components)

The amount of the other components may be 0.1 parts by weight or more, 1 part by weight or more, 3 parts by weight or more, 5 parts by weight or more, 10 parts by weight or more, 15 parts by weight or more, or 20 parts by weight or more, per 100 parts by weight of the amide group-containing polymer. The amount of the other components may be 50 parts by weight or less, 40 parts by weight or less, 30 parts by weight or less, 20 parts by weight or less, 10 parts by weight or less, or 5 parts by weight or less, per 100 parts by weight of the amide group-containing polymer.

### <Method for producing dispersion, water-repellent composition, or oil-resistant agent composition>

The method for producing the dispersion, water-repellent composition, or oil-resistant agent composition in the present disclosure includes producing an amide group-containing polymer by the above-described method for producing an amide group-containing polymer.

In order for the dispersion, water-repellent composition, or oil-resistant agent composition to have a desired composition in a reaction solution after polymerization, each may be produced by adding the above-described each solvent, each additive, or the like. A component such as an organic solvent may be partially distilled off from the reaction solution after polymerization, if necessary. For example, a monomer is polymerized in the presence of an organic solvent to produce an amide group-containing polymer followed by addition of water, and then the organic solvent may be distilled off.

### <Textile product or paper product>

The treated product in the present disclosure is a product in which a substrate is treated with the above-described water-repellent composition or oil-resistant agent composition (the "water-repellent composition or oil-resistant agent composition" is also simply referred to as a "water-repellent composition"), and the treated product is preferably a textile product or paper product.

Examples of the substrates of the textile product include animal and vegetable natural fibers such as cotton, hemp, wool, and silk; synthetic fibers such as polyamide, polyester, polyvinyl alcohol, polyacrylonitrile, polyvinyl chloride, and polypropylene; semi-synthetic fibers such as rayon and acetate; inorganic fibers such as a glass fiber, a carbon fiber, and an asbestos fiber; or mixed fibers thereof. The textile products include woven fabrics, knitted fabrics, and non-woven fabrics, fabrics in clothing form and carpets, and fibers, yarns and intermediate textile products (for example, slivers or coarse yarns) before being formed into fabrics may also undergo treatments.

Examples of the substrates of the paper products include papers made of bleached or unbleached chemical pulps such as kraft pulp or sulfite pulp; bleached or unbleached high-yield pulps such as crushed wood pulp, mechanical pulp, or thermomechanical pulp; wastepaper pulps such as waste newspaper, waste magazines, waste cardboards, or deinked wastepaper; containers made of paper, formed articles made of paper, etc. Specific examples of the paper product include food packaging paper, base paper for gypsum board, coated base paper, medium quality paper, general liner and core, neutral pure white roll paper, neutral liner, rust-proof liner and metal joined paper, kraft paper, neutral printing and writing paper, neutral coated base paper, neutral PPC paper, neutral thermal paper, neutral pressure sensitive base paper, neutral inkjet paper and neutral information paper, and molded paper (molded containers).

Examples of substrates treated with the water-repellent composition or oil-resistant agent composition of the present disclosure include not only the textile products or paper products, but also others such as stone, filters (for example, electrostatic filters), dust masks, components of fuel cells (for example, gas diffusion electrodes and gas diffusion supports), glass, wood, leather, fur, asbestos, brick, cement, metals and oxides, ceramic products, plastics, painted surfaces, and plasters.

### <Method for producing textile product or paper product>

The method for producing the textile product or paper product in the present disclosure includes producing the water-repellent composition or the oil-resistant agent composition by the above-described method for producing the water-repellent composition or oil-resistant agent composition; and treating a substrate with the water-repellent composition or the oil-resistant agent composition.

The term "treatment" refers to applying the water-repellent composition or oil-resistant agent composition to a substrate by immersing, spraying, coating, etc. The treatment allows a polymer of the water-repellent composition or oil-resistant agent composition as an active ingredient to penetrate inside of the substrate and/or to adhere to a front side of the substrate.

The water-repellent composition or oil-resistant agent composition of the present disclosure can be applied as a treatment agent (particularly a surface-treating agent) to a substrate by conventionally known methods. A method for treating the substrate may be a method that includes dispersing and diluting the water-repellent composition in the present disclosure in an organic solvent or water, if necessary, and allowing the resultant to adhere to a surface of the substrate by any known methods such as dip coating, spray coating, or foam coating, and then drying it. After drying, a textile product is obtained with a solid component in the water-repellent composition adhered. If necessary, a suitable crosslinking agent may be applied in combination with the water-repellent composition, followed by curing. Furthermore, the water-repellent composition of the present disclosure may be used in combination with various additives such as water- and/or oil-repellent agents, anti-slip agents, antistatic agents, texture modifiers, softening agents, antibacterial agents, flame retarders, coating material fixing agents, wrinkle-resistant agents, drying rate adjusters, crosslinking agents, film formation agents, compatibilizing agents, antifreeze agents, viscosity adjusters, ultraviolet absorbers, antioxidants, pH adjusters, insect repellents, and antifoaming agents. Examples of the various additives may be the same as those described in "Other components" for the water-repellent composition hereinabove. The concentration of the hydrocarbon-based water-repellent resin in the treatment agent to be brought into contact with a substrate may be appropriately changed according to applications, but may be 0.01 to 10% by weight, for example, 0.05 to 5% by weight.

Examples of the textile products that are substrates can include various products.

The water-repellent composition or oil-resistant agent composition can be applied to the textile products by any of the methods known for treating textile products (for example, fabrics) with liquids. The textile product may be immersed in a water-repellent composition or oil-resistant agent composition, or the solution may be adhered to or sprayed on the textile product. The treated textile product is dried and subjected to curing, preferably by heating, in order to develop water- and oil-repellency. The heating temperature may be, for example, 100°C to 200°C, 100°C to 170°C, or 100°C to 120°C. Favorable performance can be obtained even by heating at lowered temperatures (for example, 100°C to 140°C) in the present disclosure. In the present disclosure, the heating time may be 5 seconds to 60 minutes, for example, 30 seconds to 3 minutes. In the case of a textile product of paper, the paper may undergo coating, or a solution may be attached or sprayed onto the paper, alternatively, the paper may be mixed with a pulp slurry before papermaking to treat the paper. The treatment may be an external addition treatment or an internal addition treatment.

Alternatively, the polymer may be applied to the textile product by a cleaning method, for example, in laundry application or dry cleaning methods.

### [Pretreatment of textile product]

The textile product may be pretreated prior to treatment with the water-repellent composition of the present disclosure. When a pretreated textile product is treated with the water-repellent composition, the textile product after the treatment with the water-repellent composition has excellent fastness.

Examples of pretreatment of textile products include cationization treatments by reaction with reactive quaternary ammonium salts or the like, anionization treatments such as sulfonation, carboxylation and phosphorylation, acetylation treatment after anionization treatment, benzoylation treatment, carboxymethylation treatment, grafting treatment, tannin acid treatment, and polymer coating treatment.

The method for pretreating the textile product is not limited, and the textile product can be pretreated by any of conventionally known methods. The method for pretreatment may include dispersing a pretreatment liquid in an organic solvent or water to dilute it therewith, as necessary, and allowing the diluted pretreatment liquid to adhere to the surface of the textile product by any known methods such as dip coating, spray coating, foam coating, and the like, and then drying the resultant. The pH, temperature, etc. of the pretreatment liquid may be adjusted according to an extent of treatment desired. As an example of the method for pretreating a textile product, a method for pretreating the textile product with a hydrocarbon-based water-repellent agent will be described in detail.

The method for pretreating a textile product may include applying, to a fiber, at least one functional group (hereinafter may be referred to as "specific functional group") selected from a monovalent group represented by - SO₃M¹ wherein M¹ represents a monovalent cation, a monovalent group represented by -COOM² wherein M² represents a monovalent cation, and a monovalent group represented by -O-P(O)(OX¹)(OX²) wherein X¹ and X² each independently represent a hydrogen atom or an alkyl group having 1 to 22 carbon atoms.

Examples of M¹ include H, K, Na, or an ammonium ion which may have a substituent. Examples of M² include H, K, Na, or an ammonium ion which may have a substituent. When X¹ or X² is an alkyl group, it is preferably an alkyl group having 1 to 22 carbon atoms and more preferably an alkyl group having 4 to 12 carbon atoms.

Fibers containing the aforementioned specific functional groups (hereinafter, also referred to as "functional group-containing fibers") can be prepared, for example, by the following methods.
(i) A compound having the above specific functional group is allowed to adhere to a fiber material. The adhesion of the compound may be in a condition such that a portion of the compound and a portion of the fibers are chemically bonded as long as the above specific functional groups remain in a sufficient amount.
(ii) A fiber is provided in which the above specific functional group has been directly introduced into the material forming the fiber.

In the case of (i), for example, a functional group-containing fiber can be obtained by treating the fiber material with a pretreatment liquid containing one or more compounds having the above specific functional group, namely, by the introducing the functional group.

Raw materials of the fiber material are not limited, and examples thereof include natural fibers such as cotton, hemp, silk, and wool, semi-synthetic fibers such as rayon and acetate, synthetic fibers such as polyamides (nylon and the like), polyester, polyurethane, polypropylene, and composite fibers and blended fibers thereof. The fiber material may be in any form of fibers (tows, slivers, and the like), yarns, knitted fabrics (including interknitted fabrics), woven fabrics (including interwoven fabrics), nonwoven fabrics, papers, and the like.

In the present embodiment, in view of favorable water-repellency of the textile products to be obtained, fiber materials containing a polyamide and polyester as a raw material are preferably used, and in particular, nylons such as nylon 6 and nylon 6,6, polyesters such as polyethylene terephthalate (PET), polytrimethyl terephthalate, and polylactic acid, and mixed fibers containing these materials are preferably used.

A Phenolic polymer can be used as the compound having -SO₃M¹ described above. Examples of such a phenolic polymer include those containing at least one compound represented by the following general formula: 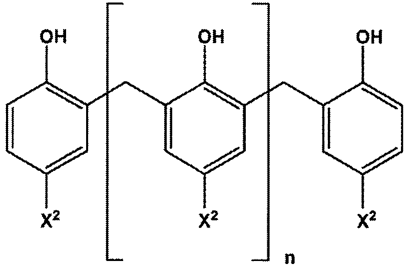
wherein in formula (2), X² represents -SO₃M³, wherein M³ represents a monovalent cation, or a group represented by the following general formula, and n is an integer of 20 to 3,000; 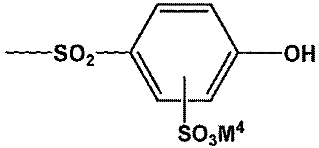
wherein M⁴ represents a monovalent cation.

Examples of M³ include H, K, Na or an ammonium ion that may have a substituent.

Examples of M⁴ include H, K, Na or an ammonium ion which may have a substituent.

The compounds represented by the general formula above may be, for example, formalin condensates of phenol sulfonic acid and formalin condensates of sulfonated bisphenol S.

Examples of the compound having -COOM² above include a polycarboxylic acid-based polymer.

The polycarboxylic acid-based polymer used can be, for example, a polymer synthesized by using acrylic acid, methacrylic acid, maleic acid, and the like as monomers by a conventionally known radical polymerization method, or a commercially available polymer.

A method for producing the polycarboxylic acid-based polymers may include, for example, adding a radical polymerization initiator to an aqueous solution of the aforementioned monomer and/or salt thereof and heating and reacting the mixture at 30 to 150°C for 2 to 5 hours. In this case, alcohols such as methanol, ethanol, and isopropyl alcohol, or aqueous solvents such as acetone may be added to the aqueous solution of the above monomer and/or salt thereof. Examples of the radical polymerization initiator include persulfates such as potassium persulfate, sodium persulfate, and ammonium persulfate; redox-based polymerization initiators such as a combination of a persulfate with sodium bisulfite; hydrogen peroxide, and water-soluble azo-based polymerization initiators. These radical polymerization initiators may be used singly or in combination of two or more thereof. Furthermore, a chain transfer agent (for example, octyl thioglycolate) may be added upon radical polymerization for the purpose of adjusting the degree of polymerization.

In addition to the aforementioned monomers, a copolymerizable monomer can be used for radical polymerization. Examples of the copolymerizable monomer include vinyl monomers such as ethylene, vinyl chloride, and vinyl acetate, acrylamide, acrylates, and methacrylates. The acrylates and methacrylates preferably have a hydrocarbon group having 1 to 3 carbon atoms that may have a substituent such as a hydroxyl group. Examples of such acrylates or methacrylates include methyl acrylate, methyl methacrylate, ethyl acrylate, ethyl methacrylate, 2-hydroxyethyl acrylate, 2-hydroxyethyl methacrylate, propyl acrylate, and propyl methacrylate. These copolymerizable monomers may be used singly or in combination of two or more thereof.

The carboxyl group in the polycarboxylic acid-based polymer may be free or neutralized with an alkali metal, an amine-type compound, or the others. Examples of the alkali metal include sodium, potassium, and lithium, and examples of the amine-type compound include ammonia, monoethanolamine, diethanolamine, and triethanolamine.

A weight average molecular weight of the polycarboxylic acid-based polymer is preferably 1,000 to 20,000 and more preferably 3,000 to 15,000 in view of favorable water-repellency of the textile product to be obtained.

Commercially available products of the polycarboxylic acid-based polymer can be used, including "NEOCRYSTAL 770" (trade name, manufactured by NICCA CHEMICAL CO., LTD.) and "CELLOPOL PC-300" (trade name, manufactured by Sanyo Chemical Industries, Ltd.).

Examples of the compound having -O-P(O)(OX¹)(OX²) as described above include phosphate ester compounds represented by the following general formula: 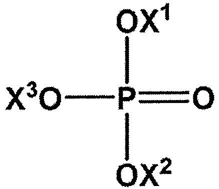 wherein, X¹ or X² is the same as defined above, and X³ represents an alkyl group having 1 to 22 carbon atoms.

As the aforementioned phosphoric acid ester compound, phosphoric acid monoesters, diesters and triesters, and mixtures thereof can be used in which the alkyl ester moiety is an alkyl group having 1 to 22 carbon atoms.

In view of favorable water-repellency of the textile products to be obtained, lauryl phosphoric acid ester and decyl phosphoric acid ester are preferably used.

As the phosphoric acid ester compound, commercially available products such as PHOSPHANOL ML-200 (trade name, manufactured by TOHO Chemical Industry Co., Ltd.) can be used.

A pretreatment liquid containing one or more of the compounds having the aforementioned specific functional group can be, for example, an aqueous solution of the compound described above. The pretreatment liquid may also contain an acid, alkali, surfactant, chelating agent, and the others.

Examples of the method for treating a fiber material with the above pretreatment liquid include padding treatment, dip treatment, spray treatment, and coating treatment. Examples of padding treatment include the method involving using the padding apparatus as described on pages 396 to 397 of Seni Sensyoku Kako Jiten (in Japanese; Fiber-dyeing process dictionary) (published by THE NIKKAN KOGYO SHIMBUN, LTD., 1963) and pages 256 to 260 of Irozome Kagaku (in Japanese; dyeing chemistry) III (published by JIKKYO SHUPPAN CO., LTD., 1975). Examples of the coating treatment include the method involving using a coating machine as described on pages 473 to 477 of Sensyoku Shiage Kiki Soran (in Japanese; Comprehensive guide to dyeing and finishing machines) (published by Fiber Japan CO., LTD., 1981). Examples of the dip treatment include the method involving using a batch type dyeing machine as described in pages 196 to 247 of Sensyoku Shiage Kiki Soran (in Japanese) (published by Fiber Japan CO., LTD., 1981), and for example, a jet dyeing machine, air flow dyeing machine, drum dyeing machine, wince dyeing machine, washer dyeing machine, and cheese dyeing machine can be used. Examples of the spray treatment includes a method involving using an air spray that nebulizes and sprays a treatment liquid by compressed air, or an air spray by hydraulic pressure nebulization system. In this case, the concentration of the treatment liquid and treatment conditions of heat treatment after application can be adjusted appropriately, taking into consideration various conditions such as their purposes and performance. Moreover, in a case in which the pretreatment liquid contains water, it is preferably dried to remove water after the pretreatment liquid has been allowed to adhere to the fiber material. The Drying method are not limited, and either a dry heat method or a wet heat method may be employed. Drying temperatures are also not limited, and for example, drying may be carried out at room temperature to 200°C for 10 seconds to several days. Heat treatment at a temperature of 100 to 180°C for about 10 seconds to 5 minutes may be carried out after the drying, as necessary.

In a case in which the textile material is those to be dyed, treatment with the pretreatment liquid may be carried out either before dyeing or in the same bath as in dyeing. However, in a case in which reduction soaping is carried out, there is a risk that the compounds having the aforementioned specific functional groups (for example, phenolic polymer compounds and the like) adsorbed may drop off in the course, and therefore, the treatment with the pretreatment liquid is preferably carried out following the reduction soaping after the dyeing.

The treatment temperature in the dip treatment can be 60 to 130°C. The treatment time can be 5 to 60 minutes.

Introducing the functional group by the pretreatment liquid is preferably treatment such that the amount of the adhering compound having the aforementioned specific functional group is 1.0 to 7.0 parts by weight, per 100 parts by weight of the fiber material. Within this range, both durable water-repellency and texture can be achieved at a high level.

The pH of the pretreatment liquid is preferably adjusted to 3 to 5. The pH adjustment can be carried out by using a pH adjuster such as acetic acid or malic acid.

A salt can be used in combination with the pretreatment liquid to adsorb the compound having the aforementioned specific functional group effectively onto the fiber material by a salting effect. Examples of the salt that can be used include sodium chloride, sodium carbonate, ammonium sulfate, and sodium sulfate.

In introducing the functional group by the pretreatment liquid, an excess amount of the compound having the aforementioned specific functional group, which has been given by the treatment, is preferably removed. Examples of the removal method include washing with water. Sufficient removal can avoid inhibition of development of water-repellency in the subsequent water-repellent treatment, and additionally, the textile product to be obtained has the favorable texture. The functional group-containing fibers to be obtained are preferably sufficiently dried before contacting them with the hydrocarbon-based water-repellent agent.

Examples of (ii) the fiber in which the aforementioned specific functional group has been introduced directly into the material forming the fiber include a cation-dyeable polyester (CD-PET).

In view of favorable water-repellency of the textile products to be obtained, the functional group-containing fiber preferably has a zeta potential of its surface of - 100 to -0.1 mV and more preferably -50 to -1 mV. The zeta potential of the fiber surface can be measured, for example, using a zeta potential and particle size measurement system, ELSZ-1000ZS (manufactured by Otsuka Electronics Co., Ltd.).

The embodiments have been described above, however, it will be understood that various changes can be made in the forms and details without departing from the gist and scope of the claims.

### Examples

Hereinbelow, Examples of the present disclosure will be illustrated in detail; however, Examples do not restrict the present disclosure.

### [Pretreatment of raw material alcohol]

### (Water washing treatment of raw material alcohol)

A commercially available raw material alcohol (hereafter referred to as an amide alcohol A), consisting of a mixture of 65 mass% stearic acid monoethanolamide (C18-OH) and 35 mass% palmitic acid monoethanolamide (C16-OH), was washed with water as follows.
1. A 1,000 cc four-necked flask is charged with 50 g of the amide alcohol A and 80 g of IPA.
2. The flask is equipped with a cooling tube and thermometer.
3. The mixture is heated to 60°C and dissolved.
4. 480 g of warm water at 60°C is gradually added.
5. Stirring is stopped and the resulting mixture is allowed to stand still for 30 minutes.
6. The above mixture is filtered under reduced pressure to collect a precipitate.
7. 100 g of warm pure water is poured into the filtered precipitate followed by washing.
8. The precipitate is dried at 110°C for 4 hours.
9. The dried precipitate is crushed and collected.

The amide alcohol after this once water washing is hereafter referred to as an amide alcohol B. A pH of the filtrate resulting from water washing was measured, exhibiting alkalinity with a value of 9.9.

The amide alcohol B further underwent water washing again in the same manner (twice water washing). The amide alcohol after this twice water washing is hereafter referred to as an amide alcohol C.

The Na content of the amide alcohols A, B, and C was measured using ICP atomic emission spectroscopy, and was found to be 1,430 ppm, 151 ppm, and 8 ppm, respectively. Additionally, no K or Li was detected.

| | Water washing | Na content (ppm) |
|---|---|---|
| Amide alcohol A | Before water washing | 1430 |
| Amide alcohol B | Once water washing | 151 |
| Amide alcohol C | Twice water washing | 8 |

### (Alkali adsorption treatment of raw material alcohol)

### •Alkali adsorption treatment 1

The amide alcohol A underwent an alkali adsorption treatment as follows.

The raw material alcohol after treatment is referred to as an amide alcohol D.
1. A 1,000 cc four-necked flask is charged with 60 g of the amide alcohol A and 540 g of IPA.
2. The flask is equipped with a cooling tube and thermometer.
3. The mixture is heated to 60°C and dissolved.
4. 0.6 g of synthetic magnesium silicate (specific surface area: 530 m²/g) was added and the obtained solution was stirred for 90 minutes.
5. This solution staying hot is filtered under pressure to remove the synthetic magnesium silicate.
6. IPA is removed from the filtrate under heating and reduced pressure.
7. The substance obtained in 6. above is crushed and collected.

### •Alkali adsorption treatment 2

The amide alcohol A was treated under the same conditions as in the alkali adsorption treatment 1 of the raw material alcohol A, except that 1.2 g of synthetic aluminum silicate (specific surface area: 610 m²/g) was added.

The raw material alcohol after treatment is referred to as an amide alcohol E.

### •Alkali adsorption treatment 3

The amide alcohol A was treated under the same conditions as in the alkali adsorption treatment 1 of the raw material alcohol A, except that 1.8 g of synthetic magnesium silicate (specific surface area: 120 m²/g) was added.

The raw material alcohol after treatment is referred to as an amide alcohol F.

The Na content of the amide alcohols D, E, and F was measured using ICP atomic emission spectroscopy, and was found to be 266 ppm, 25 ppm, and 320 ppm, respectively.

| | Alkali adsorption treatment | Na content (ppm) |
|---|---|---|
| Amide alcohol D | Treatment 1 | 266 |
| Amide alcohol E | Treatment 2 | 25 |
| Amide alcohol F | Treatment 3 | 320 |

### [Synthesis Example]

### (Synthesis Example 1)

A 200 cc four-necked flask was charged with 30 g of the amide alcohol B, 38.92 g of methyl acrylate, and 0.03 g of p-methoxyphenol. The flask was equipped with a Dean-Stark tube, a cooling tube, and a thermometer, and was immersed in an oil bath. The flask was raised to a temperature 80°C to dissolve the contents, and a sample for GC was taken. The flask was charged with 1.38 g of titanium triethanolaminate, and was raised to a temperature of 90°C under air flow to start a reaction. The reduction rate of the amide alcohol B was tracked by GC, and the reaction was continued until the conversion ratio reached 93% or more. Thereafter, the flask was lowered to a temperature of 80°C, added with 0.4 g of pure water, and stirred for 1 hour. The contents were transferred to an eggplant flask, and excess methyl acrylate was removed using an evaporator under heating and reduced pressure to obtain a solid matter. ¹H-NMR analysis confirmed the presence of a mixture of stearic acid amidoethyl acrylate and palmitic acid amidoethyl acrylate.

### (Synthesis Examples 2 to 4)

Each synthesis reaction was carried out in the same charging amounts of the raw materials excluding the raw material alcohol and in the same manner as in Synthesis Example 1, except that the raw material alcohol shown in Table 1 was used.

### (Comparative Synthesis Examples 1 and 2)

Each synthesis reaction was carried out in the same charging amounts of the raw materials excluding the raw material alcohol and in the same manner as in Synthesis Example 1, except that the raw material alcohol shown in Table 1 was used.

**Table 1**

| Synthesis | Raw material alcohol | Na amount (ppm) in raw material alcohol | Catalyst |
|---|---|---|---|
| Synthesis Ex.1 | Amide alcohol B | 151 | Titanium triethanolaminate |
| Synthesis Ex.2 | Amide alcohol C | 8 | Titanium triethanolaminate |
| Synthesis Ex.3 | Amide alcohol D | 266 | Titanium triethanolaminate |
| Synthesis Ex.4 | Amide alcohol E | 25 | Titanium triethanolaminate |
| Com. Syn. Ex.1 | Amide alcohol F | 320 | Titanium triethanolaminate |
| Com. Syn. Ex.2 | Amide alcohol A | 1430 | Titanium triethanolaminate |

Table 2 shows the results of the tracked conversion ratio to the amide group-containing monomer in Synthesis Examples 1 to 4 and Comparative Synthesis Examples 1 and 2 by GC. Table 3 also shows the LCMS analysis results of the products in Synthesis Examples 1 to 4 and Comparative Synthesis Example 1.

**Table 2. Conversion ratio (%) to amide group-containing acrylate**

| | After 3 hours | After 6 hours | After 10 hours | After 15 hours | After 20 hours |
|---|---|---|---|---|---|
| Synthesis Ex.1 | 21.3 | 45.6 | 67.5 | 87.8 | 94.4 |
| Synthesis Ex.2 | 40.1 | 68.4 | 86.3 | 94.8 | 98.1 |
| Synthesis Ex.3 | 18.6 | 43.2 | 64.7 | 84.2 | 93.1 |
| Synthesis Ex.4 | 35.4 | 64.2 | 80.1 | 94.2 | 97.6 |
| Com. Syn. Ex.1 | 17.5 | 36.3 | 58.7 | 80.4 | 89.6 |
| Com. Syn. Ex.2 | 5.9 | 13 | 21.8 | 29.5 | 33.8 |

**Table 3. LCMS analysis results of product (%)**

| | Monomer *1 | Raw material alcohol | Amide ester *2 | Michael addition product *3 |
|---|---|---|---|---|
| Synthesis Ex. 1 | 96.0 | 3.1 | 0.2 | 0.7 |
| Synthesis Ex.2 | 98.1 | 1.6 | 0.2 | 0.1 |
| Synthesis Ex.3 | 95.3 | 3.5 | 0.3 | 0.9 |
| Synthesis Ex.4 | 97.8 | 1.9 | 0.2 | 0.1 |
| Com. Syn. Ex.1 | 93.2 | 4 | 0.4 | 2.4 |

| | | | | |
|---|---|---|---|---|
| *1: Total percentage (%) by weight of palmitic acid amidoethyl acrylate (C16M) and stearic acid amidoethyl acrylate (C18M) *2: A compound corresponding to a structure in which a hydroxy group of C16-OH or C18-OH that is an impurity contained in the raw material alcohol and a carboxylic acid group of palmitic acid or stearic acid, were esterified. *3: The total percentage (%) by weight of the following compounds: A compound in which methanol was added to C16M or C18M A compound in which C18-OH or C16-OH was added to C16M or C18M A compound in which C18-OH or C16-OH was added to a dimer of C16M or C18M | | | | |

### [Polymerization Example]

### (Polymerization Example 1)

A 500 ml plastic container was charged with 30 g of a water-soluble glycol-based solvent, 30 g of the amide group-containing monomer of Synthesis Example 1, 49 g of stearyl acrylate, 1 g of N-methylol acrylamide, 180 g of pure water, 2 g of a cationic emulsifier, 2 g of sorbitan fatty acid ester, and 6 g of a polyoxyethylene alkyl ether, and the mixture was heated to 80°C, stirred with a homomixer at 2,000 rpm for 1 minute, and then emulsified and dispersed with ultrasonic waves for 15 minutes. The emulsified dispersion was transferred to a 500 ml autoclave, and after nitrogen substitution, 0.2 g of lauryl mercaptan and 20 g of vinyl chloride were charged. 1 g of 2,2-azobis(2-amidinopropane) dihydrochloride was further added, and the mixture was reacted at 60°C for 4 hours to obtain an aqueous dispersion of a polymer. This dispersion was further diluted with pure water to prepare a water dispersion 1 with a solid content concentration of 30%.

### (Polymerization Example 2)

A polymerization reaction was carried out in the same charge composition and in the same manner as in Polymerization Example 1, except that the amide group-containing monomer of Synthesis Example 2 was used, and the obtained dispersion was diluted with pure water to prepare a water dispersion 2 with a solid content concentration of 30%.

### (Polymerization Example 3)

A polymerization reaction was carried out in the same charge composition and in the same manner as in Polymerization Example 1, except that the amide group-containing monomer of Synthesis Example 3 was used, and the obtained dispersion was diluted with pure water to prepare a water dispersion 3 with a solid content concentration of 30%.

### (Polymerization Example 4)

A polymerization reaction was carried out in the same charge composition and in the same manner as in Polymerization Example 1, except that the amide group-containing monomer of Synthesis Example 4 was used, and the obtained dispersion was diluted with pure water to prepare a water dispersion 4 with a solid content concentration of 30%.

### (Comparative Polymerization Example 1)

A polymerization reaction was carried out in the same charge composition and in the same manner as in Polymerization Example 1, except that the amide group-containing monomer of Comparative Synthesis Example 1 was used, and the obtained dispersion was diluted with pure water to prepare a comparative water dispersion 1 with a solid content concentration of 30%.

### (Polymerization Example 5)

A 200 cc four-necked flask equipped with a nitrogen inlet pipe, thermometer, stirring bar, and reflux pipe was charged with 39 g of the amide group-containing monomer of Synthesis Example 1, 8 g of hydroxyethyl acrylate, 3 g of dimethylaminoethyl methacrylate, and 50 g of methyl ethyl ketone, and was stirred for 30 minutes at 60°C under a nitrogen flow. Thereafter, the flask was added with 0.5 g of azobisisobutyronitrile and was raised to a temperature of 80°C, to implement a polymerization reaction for 10 hours. To the resulting copolymer solution was added 1.7 g of acetic acid and 10 g of pure water and stirred for 30 minutes. The contents were transferred to a 1 L eggplant flask, which was further added with 223 g of pure water, and methyl ethyl ketone that was a solvent was then removed under heating and reduced pressure to obtain a water dispersion. At this time, water was also partially removed together with methyl ethyl ketone. The resulting water dispersion was further diluted with pure water to obtain a water dispersion 5 with a solid content concentration of 15%.

### (Polymerization Example 6)

A polymerization reaction was carried out in the same charge composition and in the same manner as in Polymerization Example 5, except that the amide group-containing monomer of Synthesis Example 2 was used, and the obtained dispersion was diluted with pure water to prepare a water dispersion 6 with a solid content concentration of 15%.

### (Comparative Polymerization Example 2)

A polymerization reaction was carried out in the same charge composition and in the same manner as in Polymerization Example 5, except that the amide group-containing monomer of Comparative Synthesis Example 1 was used, and the obtained dispersion was diluted with pure water to prepare a comparative water dispersion 2 with a solid content concentration of 15%.

### [Test Example]

### (Textile fabric treatment test)

### •Test Example 1

The water dispersion 1 with a solid content concentration of 30%, which was prepared in Polymerization Example 1, was further diluted with tap water to prepare treatment liquid with a solid content concentration of 1%. A polyester fabric (gray) and a nylon fabric (black) were immersed in this treatment liquid and then squeezed with a mangle. The wet pickup value was approximately 55% (polyester fabric) and approximately 35% (nylon fabric), respectively. The treated fabrics were passed through a pin tenter at 170°C for 1 minute, dried and cured. The test fabrics treated in such a manner were evaluated for water-repellency by a water-repellency test using the spray method of JIS L-1092. Table 4 shows the results of their water-repellency. In addition, Table 4 similarly shows the results of water-repellency evaluation of the test fabrics, which were selected 10 times and then dried in a tumbler at 60°C for 30 minutes according to JIS L-0217 103.

### •Test Examples 2 to 4

Each of the water dispersions 2 to 4 with a solid content concentration of 30%, prepared in Polymerization Examples 2 to 4 was diluted with tap water to a solid content concentration of 1% as in Test Example 1 to prepare treatment liquid. Using this treatment liquid, fabrics were treated as in Test Example 1 to carry out a water-repellency test, the results of which are shown in Table 4.

### •Comparative Test Example 1

The comparative water dispersion with a solid content concentration of 30%, prepared in Comparative Polymerization Example 1 was diluted with tap water to a solid content concentration of 1% as in Test Example 1 to prepare treatment liquid. Using this treatment liquid, fabrics were treated as in Test Example 1 to carry out a water-repellency test, the results of which are shown in Table 4.

**Table 4. Water-repellency**

| | | | Test Example 1 | Test Example 2 | Test Example 3 | Test Example 4 | Comp. Test Example 1 |
|---|---|---|---|---|---|---|---|
| Water-repellency | Polyester fabric | Before washing | 100 | 100 | 100 | 100 | 100 |
| | | After ten-time washing | 90 | 90 | 90 | 90 | 90 |
| | Nylon fabric | Before washing | 100 | 100 | 100 | 100 | 100 |
| | | After ten-time washing | 80 | 80 | 80 | 80 | 80 |

### (Stability test)

### •Stability Test Examples 1 to 4

### •Spontaneous sedimentation test at 40°C

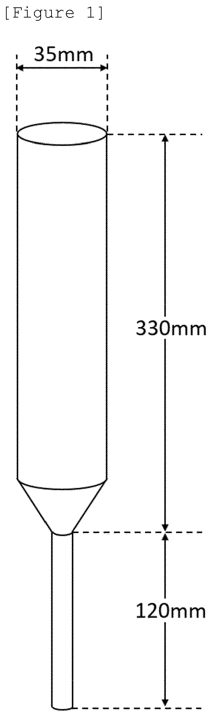

A thin-tapered sedimentation tube with a diameter of 35 mm (the diameter of the thin-tapered portion is 8 mm) shown in Figure 1 was charged with 200 ml of each of the water dispersions 1 to 4, prepared in Polymerization Examples 1 to 4, and stored at 40°C for one month under an atmosphere, and the amount of sedimentation was then measured, the results of which are shown in Table 5. Herein, the amount of sedimentation refers to a height (mm) of a sedimented portion accumulated at the bottom.

### •Centrifugal sedimentation test

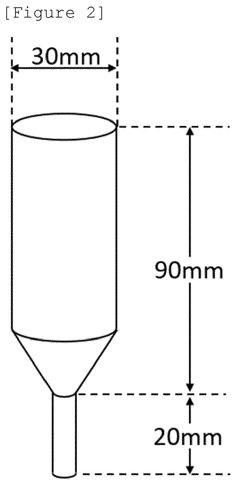

A thin-tapered centrifugal sedimentation tube with a diameter of 30 mm (the diameter of the thin-tapered portion is 8 mm) shown in Figure 2 was also charged with 30 g of each of the water dispersions 1 to 4, prepared in Polymerization Examples 1 to 4, and the thin-tapered centrifugal sedimentation tube with a rubber tube set at the thin-tapered portion was rotated at 1,000 rpm for 30 minutes and was then further rotated at 2,000 rpm for another 30 minutes with a centrifugal separator, and the amount of sedimentation was then measured, the results of which are shown in Table 5. Herein, the amount of sedimentation refers to a height (mm) of a sedimented portion accumulated at the bottom.

### •Comparative Stability Test Example 1

Table 5 shows the results that the comparative water dispersion 1 with a solid content concentration of 30%, prepared in Comparative Polymerization Example 1, was subjected to the spontaneous sedimentation test and centrifugal sedimentation test at 40°C in the same manner as in Stability Test Examples 1 to 4.

### •Stability Test Examples 5 and 6

Table 5 shows the results that the water dispersions 5 and 6 with a solid content concentration of 15%, were subjected to the spontaneous sedimentation test and centrifugal sedimentation test at 40°C in the same manner as in Stability Test Examples 1 to 4.

### •Comparative Stability Test Example 2

Table 5 shows the results that the comparative water dispersion 2 with a solid content concentration of 15%, was subjected to the spontaneous sedimentation test and centrifugal sedimentation test at 40°C in the same manner as in Stability Test Examples 1 to 4.

**Table 5. Stability test**

| | Stability Test Ex.1 | Stability Test Ex.2 | Stability Test Ex.3 | Stability Test Ex.4 | Com. Stability Test Ex.1 | Stability Test Ex.5 | Stability Test Ex.6 | Comp. Stability Test Ex.2 |
|---|---|---|---|---|---|---|---|---|
| Spontaneous sedimentation test at 40°C (mm) | 1 | 0.5 | 2 | 0.5 | 8 | 0.5 | 0.2 | 7 |
| Centrifugal sedimentation test (mm) | 0.1 | 0 | 0.2 | 0 | 2 | 0.1 | 0 | 1.5 |

### (Paper treatment test)

### •Test Example 5

As wool pulp, a pulp slurry with weight ratios of LBKP (hardwood bleached kraft pulp) and NBKP (softwood bleached kraft pulp) being 60% by weight and 40% by weight, respectively, and a freeness of 400 ml (Canadian Standard Freeness), was prepared, and the pulp slurry was added with a wet paper strengthening agent and sizing agent to treat and obtain paper with a paper density of 0.58 g/cm³ and a basis weight of 45 g/m², using a long-net papermaking machine. The oil resistance (KIT value) of this paper was zero and its water resistance (Cobb value) was 52 g/m². The paper was subjected to the following size press treatment.

First, dilution with water was performed such that the solid content concentration of the water dispersion 5 obtained in Polymerization Example 5 was 2.4% by weight and the concentration of hydroxyethylated starch was 7% by weight to thereby prepare treatment liquid. Next, the paper was treated with the above-described treatment liquid in a size press machine and dried in a drum dryer to obtain oil-resistant paper (processed paper). The coating amount of hydroxyethylated starch and the copolymer in the resulting oil-resistant paper was 1.1 g/m² (of the total amount, the coating amount of the copolymer was 0.28 g/m²). The evaluation results of the oil resistance (KIT value) and water resistance (Cobb value) of the resulting oil-resistant paper are shown in Table 6.

### •Test Example 6

Paper was treated in the same manner as in Test Example 5, except that the water dispersion 6 obtained in Polymerization Example 6 was used to obtain oil-resistant paper (processed paper). The evaluation results of the oil resistance (KIT value) and water resistance (Cobb value) of the obtained oil-resistant paper are shown in Table 6.

### •Comparative Test Example 2

Paper was treated in the same manner as in Test Example 5, except that the comparative water dispersion 2 obtained in Comparative Polymerization Example 2 was used to obtain oil-resistant paper (processed paper). The evaluation results of the oil resistance (KIT value) and water resistance (Cobb value) of the obtained oil-resistant paper are shown in Table 6.

**Table 6**

| | Test Ex.5 | Test Ex.6 | Comp. Test Ex. 2 |
|---|---|---|---|
| Oil resistance (KIT value) | 5 | 5 | 5 |
| Water resistance (Cobb value) | 21 | 20 | 20 |

## Claims

1. A method for producing an amide group-containing monomer comprising
(I) reducing amounts of Na, K, and Li present as impurities in an amide group-containing alcohol (2) of the formula HO-Y¹-NHC(=O)-R¹² wherein Y¹ and R¹² are as defined below;
(II) conducting a transesterification reaction to obtain an amide group-containing monomer (1) of the formula:
CH₂=C(-R¹¹)C(=O)O-Y¹-NHC(=O)-R¹²
wherein
R¹¹ is H, halogen or a monovalent organic group selected from the group consisting of a methyl group, a cyano group, a substituted or unsubstituted benzyl group, or a substituted or unsubstituted phenyl group,
Y¹ is a divalent C₁₋₆-hydrocarbon group, and
R¹² is a monovalent C₆₋₄₀-hydrocarbon group,
from the amide group-containing alcohol (2),
wherein the total amount of Na, K, and Li present as impurities in the alcohol (2) is ≤ 300 ppm.

2. The method of claim 1, wherein the total amount of alkali metal elements present as impurities in the alcohol (2) is ≤ 300 ppm.

3. The method of claim 1 or 2, wherein reducing amounts of Na, K, and Li present as impurities in the alcohol (2) is conducted by water washing or alkali adsorption treatment.

4. The method of any of claims 1-3, wherein in the alcohol (2) R¹² is an aliphatic C_{≥11}-hydrocarbon group.

5. The method of any of claims 1-4, wherein in the alcohol (2) R¹¹ is H or methyl, Y¹ is C₂₋₄-alkylene, and R¹² is linear C₁₀₋₃₀-alkyl.

6. The method of any of claims 1-5, wherein the total amount of Na, K and Li present as impurities in the alcohol (2) is ≤ 30 ppm.

7. The method of any of claims 1-6,
wherein the amount of a Michael addition product present as an impurity in the amide group-containing monomer (1) is ≤ 2 wt.%.

8. The method of any one of claims 1-7, wherein R¹² is an aliphatic C_{≥11}-hydrocarbon group.

9. The method of any one of claims 1-8, wherein R¹¹ is H or methyl, Y¹ is C₂₋₄-alkylene, and R¹² is linear C₁₀₋₃₀-alkyl.

10. A method for producing an amide group-containing polymer, comprising conducting the method of any of claims 1-9 and then polymerizing an amide group-containing monomer so obtained.

11. The method of claim 10, wherein, in the polymerizing, at least one selected from a hydrocarbon-based monomer, a crosslinkable monomer, and a halogenated olefin is copolymerized.

12. A method for producing a dispersion, comprising producing an amide group-containing polymer by the method of claim 10 or 11.

13. A method for producing a composition, which is a water-repellent composition or an oil-resistant agent composition, comprising producing an amide group-containing polymer by the method of claim 10 or 11.

14. A method for producing a treated textile product or paper product, comprising producing a composition by the method of claim 13; and treating a substrate with said composition.

## Patentansprüche

1. Verfahren zum Herstellen eines amidgruppenhaltigen Monomers, umfassend
(I) Reduzieren der Mengen an Na, K und Li, die als Verunreinigungen in einem amidgruppenhaltigen Alkohol (2) der Formel HO-Y¹-NHC(=O)-R¹² vorliegen, wobei Y¹ und R¹² wie nachstehend definiert sind;
(II) Durchführen einer Umesterungsreaktion zum Erhalten eines amidgruppenhaltigen Monomers (1) der Formel:
CH₂=C(-R¹¹)C(=O)O-Y¹-NHC(=O)-R¹²
wobei
R¹¹ H, ein Halogen oder eine einwertige organische Gruppe ist, die ausgewählt ist aus der Gruppe, bestehend aus einer Methylgruppe, einer Cyanogruppe, einer substituierten oder unsubstituierten Benzylgruppe oder einer substituierten oder unsubstituierten Phenylgruppe,
Y¹ eine zweiwertige C₁₋₆-Kohlenwasserstoffgruppe ist, und
R¹² eine einwertige C₆₋₄₀-Kohlenwasserstoffgruppe aus dem amidgruppenhaltigen Alkohol (2) ist, wobei die Gesamtmenge an Na, K und Li, die als Verunreinigungen in dem Alkohol (2) vorhanden sind, ≤ 300 ppm beträgt.

2. Verfahren nach Anspruch 1, wobei die Gesamtmenge der in dem Alkohol (2) als Verunreinigungen vorhandenen Alkalimetallelemente ≤ 300 ppm beträgt.

3. Verfahren nach Anspruch 1 oder 2, wobei das Reduzieren der Mengen an Na, K und Li, die als Verunreinigungen in dem Alkohol (2) vorhanden sind, durch Waschen mit Wasser oder eine Alkali-Adsorptionsbehandlung durchgeführt wird.

4. Verfahren nach einem der Ansprüche 1-3, wobei R¹² in dem Alkohol (2) eine aliphatische C_{≥11}-Kohlenwasserstoffgruppe ist.

5. Verfahren nach einem der Ansprüche 1-4, wobei in dem Alkohol (2) R¹¹ H oder Methyl ist, Y¹ C₂₋₄-Alkylen ist und R¹² lineares C₁₀₋₃₀-Alkyl ist.

6. Verfahren nach einem der Ansprüche 1-5, wobei die Gesamtmenge an Na, K und Li, die als Verunreinigungen in dem Alkohol (2) vorhanden sind, ≤ 30 ppm beträgt.

7. Verfahren nach einem der Ansprüche 1-6,
wobei der Gehalt an einem Michael-Additionsprodukt, das als eine Verunreinigung in dem amidgruppenhaltigen Monomer (1) vorliegt, ≤ 2 Gew.-% beträgt.

8. Verfahren nach einem der Ansprüche 1-7, wobei R¹² eine aliphatische C_{≥11}-Kohlenwasserstoffgruppe ist.

9. Verfahren nach einem der Ansprüche 1-8, wobei R¹¹ H oder Methyl ist, Y¹ C₂₋₄-Alkylen ist und R¹² lineares C₁₀₋₃₀-Alkyl ist.

10. Verfahren zum Herstellen eines amidgruppenhaltigen Polymers, umfassend das Durchführen des Verfahrens nach einem der Ansprüche 1-9 und dann das Polymerisieren eines so erhaltenen amidgruppenhaltigen Monomers.

11. Verfahren nach Anspruch 10, wobei bei der Polymerisation mindestens eines, ausgewählt aus einem Monomer auf Kohlenwasserstoffbasis, einem vernetzbaren Monomer und einem halogenierten Olefin, co-polymerisiert wird.

12. Verfahren zum Herstellen einer Dispersion, umfassend das Herstellen eines amidgruppenhaltigen Polymers durch das Verfahren nach Anspruch 10 oder 11.

13. Verfahren zum Herstellen einer Zusammensetzung, bei der es sich um eine wasserabweisende Zusammensetzung oder eine ölbeständige Mittelzusammensetzung handelt, umfassend das Herstellen eines amidgruppenhaltigen Polymers durch das Verfahren nach Anspruch 10 oder 11.

14. Verfahren zum Herstellen eines behandelten Textilprodukts oder Papierprodukts, umfassend das Herstellen einer Zusammensetzung durch das Verfahren nach Anspruch 13; und das Behandeln eines Substrats mit dieser Zusammensetzung.

## Revendications

1. Procédé pour produire un monomère contenant un groupe amide comprenant
(I) la réduction des quantités de Na, K et Li présents en tant qu'impuretés dans un alcool (2) contenant un groupe amide de formule HO-Y¹-NHC(=O)-R¹² dans lequel Y¹ et R¹² sont tels que définis ci-dessous ;
(II) la réalisation d'une réaction de transestérification pour obtenir un monomère (1) contenant un groupe amide de formule :
CH₂=C(-R¹¹)C(=O)O-Y¹-NHC(=O)-R¹²
dans lequel
R¹¹ est H, un halogène ou un groupe organique monovalent sélectionné dans le groupe consistant en un groupe méthyle, un groupe cyano, un groupe benzyle substitué ou non substitué, ou un groupe phényle substitué ou non substitué,
Y¹ est un groupe hydrocarboné divalent en C₁₋₆, et
R¹² est un groupe hydrocarboné monovalent en C₆₋₄₀, provenant de l'alcool (2) contenant un groupe amide, dans lequel la quantité totale de Na, K et Li présents en tant qu'impuretés dans l'alcool (2) est ≤ 300 ppm.

2. Procédé selon la revendication 1, dans lequel la quantité totale d'éléments alcalins métalliques présents en tant qu'impuretés dans l'alcool (2) est ≤ 300 ppm.

3. Procédé selon la revendication 1 ou la revendication 2, dans lequel la réduction des quantités de Na, K et Li présents en tant qu'impuretés dans l'alcool (2) est réalisée par lavage à l'eau ou traitement par adsorption alcaline.

4. Procédé selon l'une quelconque des revendications 1 à 3, dans lequel dans l'alcool (2) R¹² est un groupe hydrocarboné aliphatique en C_{≥11}.

5. Procédé selon l'une quelconque des revendications 1 à 4, dans lequel dans l'alcool (2) R¹¹ est H ou méthyle, Y¹ est alkylène en C₂₋₄, et R¹² est alkyle linéaire en C₁₀₋₃₀.

6. Procédé selon l'une quelconque des revendications 1 à 5, dans lequel la quantité totale de Na, K et Li présents en tant qu'impuretés dans l'alcool (2) est ≤ 30 ppm.

7. Procédé selon l'une quelconque des revendications 1 à 6,
dans lequel la quantité d'un produit d'addition de Michael présent en tant qu'impureté dans le monomère (1) contenant un groupe amide est ≤ 2 % en poids.

8. Procédé selon l'une quelconque des revendications 1 à 7, dans lequel R¹² est un groupe hydrocarboné aliphatique en C_{≥11}.

9. Procédé selon l'une quelconque des revendications 1 à 8, dans lequel R¹¹ est H ou méthyle, Y¹ est alkylène en C₂₋₄, et R¹² est alkyle linéaire en C₁₀₋₃₀.

10. Procédé pour produire un polymère contenant un groupe amide, comprenant la réalisation du procédé selon l'une quelconque des revendications 1 à 9, puis la polymérisation d'un monomère contenant un groupe amide ainsi obtenu.

11. Procédé selon la revendication 10, dans lequel, dans la polymérisation, au moins un(e) sélectionné(e) parmi un monomère à base d'hydrocarbure, un monomère réticulable, et une oléfine halogénée est copolymérisé(e).

12. Procédé pour produire une dispersion, comprenant la production d'un polymère contenant un groupe amide par le procédé selon la revendication 10 ou la revendication 11.

13. Procédé pour produire une composition, qui est une composition hydrofuge ou une composition d'agent résistant à l'huile, comprenant la production d'un polymère contenant un groupe amide par le procédé selon la revendication 10 ou la revendication 11.

14. Procédé pour produire un produit textile ou produit papier traité, comprenant la production d'une composition par le procédé selon la revendication 13 ; et le traitement d'un substrat avec ladite composition.
